# EUROPEAN PATENT SPECIFICATION

(11) **EP 4 479 412 B1**
(45) Date of publication and mention of the grant of the patent: **10.12.2025**
(21) Application number: 23715589.0
(22) Date of filing: 17.02.2023
(51) Int. Cl.: C07J 31/00, A61P 3/06, A61P 39/06, A61K 31/568

(54) **DHEA-DERIVED STEROIDS**
VON DHEA ABGELEITETE STEROIDE
STÉROÏDES DÉRIVÉS DE DHEA

(30) Priority: 17.02.2022 HU 2200041
(43) Date of publication of application: 25.12.2024
(73) Proprietor: Debreceni Egyetem, 4032 Debrecen (HU)
(72) Inventor: BÉKÉSI, Gábor, 2600 Vác (HU); BALLA, György, 4032 Debrecen (HU); BALLA, József, 4032 Debrecen (HU); POTOR, László, 4031 Debrecen (HU); NAGY, Endre, 8200 Veszprém (HU); KOVÁCS, Sándor, 8200 Veszprém (HU); PÁZMÁNY, Tamás, 1125 Budapest (HU)
(74) Representative: Danubia Patent & Law Office LLC
(86) International application number: PCT/HU2023/050005
(87) International publication number: WO 2023/156803

(56) References cited:
- WO-A2-99/63974
- GB-A- 2 208 473
- KUIPER ET AL: "Comparison of the ligand Binding Specificity and Transcript Distribution of Estrogen Receptors alpha and beta", ENDOCRINOLOGY, THE ENDOCRINE SOCIETY, US, vol. 138, no. 3, 1 March 1997 (1997-03-01), pages 863 - 870, XP002126536, ISSN: 0013-7227, DOI: 10.1210/EN.138.3.863
- MARTYN P ET AL: "Long-chain fatty acid esters of 5-androstene-3@b,17@b-diol: Composition and turnover in human mammary cancer cells in culture", STEROIDS, ELSEVIER SCIENCE PUBLISHERS, NEW YORK, NY, US, vol. 54, no. 2, 1 August 1989 (1989-08-01), pages 245 - 255, XP023431739, ISSN: 0039-128X, [retrieved on 19890801], DOI: 10.1016/0039-128X(89)90097-4
- SONG CHING ET AL: "Auto-oxidized cholesterol sulfates are antagonistic ligands of liver X receptors: implications for the development and treatment of atherosclerosis", STEROIDS, vol. 66, no. 6, 1 June 2001 (2001-06-01), US, pages 473 - 479, XP055840457, ISSN: 0039-128X, DOI: 10.1016/S0039-128X(00)00239-7

## Description

### FIELD OF THE INVENTION

The invention relates to a novel steroid for use against arteriosclerosis and/or atherosclerosis as well as pharmaceutical compositions and medical uses thereof.

### BACKGROUND ART

Atherosclerosis is an inflammatory disease associated with hardening of the arteries, which can cause a variety of consequences including chronic kidney disease (CKD), heart attacks, stroke or thrombosis. According to WHO (World Health Organization), atherosclerosis is a considerable cause of mortality and morbidity worldwide (31%). The progression of atherosclerosis is linked to the development of calcification of heart valves called calcific aortic valve disease (CAVD). It is demonstrated by multiple studies that CAVD is accompanied by lipid accumulation, inflammation, and calcification resulting within the heart valvular tissue [Lusis AJ, *et al.* 2004; Mohler ER, 2004; Speer MY, *et al.* 2004; Mohler ER, 2001; Chester A. 2011]. Nowadays, CAVD is a cardinal problem as pharmacology drugs against aortic valves calcification are not available [Yutzey KE, *et al.* 2014].

Békési *et al.* have found that sex steroids and cortisol significantly reduced the release of superoxide anion from human neutrophils [Békési *et al.* 2000].

Brancaleone V. *et al.* evaluated H₂S biosynthesis in rat isolated aortic rings following androgen receptor stimulation and found that H₂S biosynthesis in the rat aorta is modulated by androgen hormones but is not triggered by female hormones 17-beta-ostradiol or progesterone. They conclude that androgens can exert protective actions on cardiovascular and metabolic functions by triggering a variety of beneficial effects mediated by H₂S [Brancaleone *et al.* 2015].

Naftolin F *et al.* have shown that estradiol-induced neural cell adhesion molecule (nCAM) sialylases are present in vascular endothelial cells and that pretreatment of human arterial endothelial cells with estradiol, testosterone, dehydroepiandrosterone and dihydrotestosterone all induced sialylation of endothelial cells, reduced the capture of monocytes and are protective against atherogenesis and its sequellae [Naftolin F *et al.* 2016].

Dehydroepiandrosterone (DHEA) was found in early studies already in the '90s to inhibit iron dependent lipid peroxidation and to enhance the superoxide dismutase generation in hepatic mitochondria, and that it is preventive in the development of atherosclerosis [Schauer JE *et al.* 1990; Aragno M. *et al.* 1993].

Magyar *et al.* have studied the role of sulphatation-desulphatation of DHEA/DHEAS (dehydroepiandrosterone sulphate) in Sprague-Dawley adult male rats *in vivo* by steroid sulphotransferases and sulphatases and found that DHEAS had a more pronounced effect than DHEA on total scavenger capacity (TSC) [Magyar *et al.* 2011]. Furthermore, GB-A-2,208,473 and WO-99/63974 disclose DHEA sulphate (DHEAS) for the treatment of cardiovascular diseases.

Hochberg RB *et al.* studied and reviewed already in the early '90s the role of steroidal fatty acid esters and reported that androsterone esters have been found in human breast tumors and breast cyst fluid [Hochberg *et al.* 1991].

A decade later Loria, Roger M. suggested that 5-androstene-3beta,17alpha-diol and possibly its esters are inhibitors of tumor growth. Otherwise, the literature of such ester derivatives is sparse [Loria, Roger M., US 2001/0014675 A1].

Song, Ching *et al.* [Song *et al.* 2001] teaches auto-oxidized cholesterol sulfates are antagonistic ligands of liver X receptors and suggest that since LXR agonists can counteract the activity of these antagonists they may have therapeutic potential against atherosclerosis. However, the authors have found that non-sulfated forms of these compounds as well as many other steroid sulfates, including sulfate of 5-androsten-3β,17β-diol, had no significant effect, contrary to 5α,6α-epoxycholesterol.

It is to be mentioned that the administration of steroids is counter-indicated in human medicine because, besides the antioxidant effect, they also have strong endocrinological effect which is clearly undesired in case of an anti-atherogenic medicament.

Moreover, the present inventors have found that in animal experiments neither DHEA nor DHEAS (dehydroepiandrosterone sulphate) had actual positive effect against atherosclerosis whereas both compounds proved to be toxic and induced mortality.

The present inventors have unexpectedly found that a narrow range of alkanoyl esters of DHEAS prevent and treat atherosclerotic plaque formation and calcification in ApoE^{-/-} mice fed with atherogenic diet. Therefore, DHEA-derived steroid represents a novel potential treatment preventing and/or reversing atherosclerosis and valvular calcification.

### BRIEF DESCRIPTION OF THE INVENTION

### Compounds

The invention relates to compounds according to formula (I), 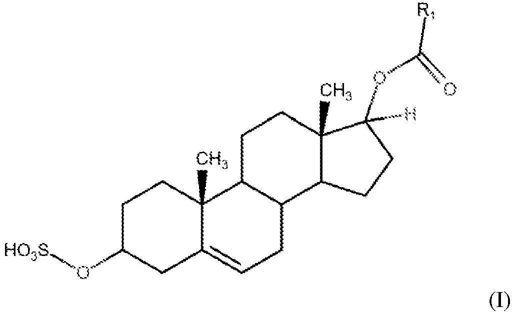 wherein R₁ is C₇-C₁₃ alkyl,
or any salt or solvate thereof, preferably any pharmaceutically acceptable salt thereof.

Preferably the C₇-C₁₃ alkyl is n-alkyl, i.e. a linear alkyl.

Preferably, R₁ is C₉-C₁₃ alkyl.

Particularly preferably, R₁ is C₇₋C₁₁ alkyl.

Particularly preferably, R₁ is C₁₁-C₁₃ alkyl.

In a further embodiment, R₁ is a C₈-C₁₂ or a C₉-C₁₂ alkyl.

In a further embodiment, R₁ is a C₈-C₁₁ or a C₉-C₁₁ alkyl.

In an embodiment, R₁ is C₁₀-C₁₂ alkyl.

More particularly preferably, R₁ is C₁₁ alkyl.

In a preferred embodiment, R₁ is any alkyl defined above. Preferably R₁ is n-alkyl.

Highly preferably, R₁ is C₁₁ n-alkyl, i.e. the compound is 17-lauroyloxy-5-androstene-3-sulphate.

Preferably the salt is sodium salt or potassium salt, in particular sodium salt.

Preferably the alkyl is n-alkyl, i.e. a linear alkyl.

Preferably the salt is sodium salt. Preferably the solvate is water solvate.

In an embodiment the configuration at carbon 3 is 3β.

In an embodiment the configuration at carbon 17 is 17β.

In a preferred embodiment the configuration is 3β,17β.

In an embodiment the configuration at carbon 3 is 3α.

In an embodiment the configuration at carbon 17 is 17α.

In an embodiment any of the compounds of general formula (I) has general formula (Is1)

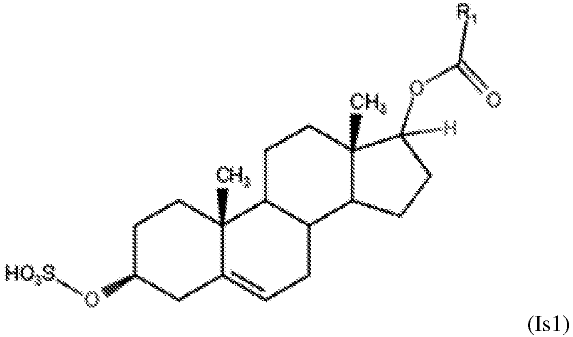

The invention relates to any of the compounds according to the invention for use in the treatment of arteriosclerosis, in particular atherosclerosis in a subject. The subject is a vertebrate having a blood circulation system (cardiovascular system or the vascular system), e.g. a mammalian subject as defined herein. The mammalian subject is preferably a human subject.

The invention relates to any of the compounds according to the invention for use in the prevention or reducing the risk of atherosclerosis.

The invention relates to any of the compounds according to the invention for use in the prevention of or in reducing the formation of or in alleviating the formation of or in reducing the risk of atherosclerotic lesions in arteries.

The invention relates to any of the compounds according to the invention for use in the prevention of or in reducing the formation of or in alleviating the formation of or in reducing the risk of atherosclerotic plaques in arteries.

The invention relates to any of the compounds according to the invention for use in the treatment of atherosclerosis.

The invention relates to any of the compounds according to the invention for use in the therapeutic treatment of atherosclerosis. The invention relates to any of the compounds according to the invention for use in the reversion of atherosclerosis or reversion of the formation of atherosclerotic lesions in arteries.

The invention relates to any of the compounds according to the invention for use in the reversion of atherosclerosis or of the formation of atherosclerotic plaques in arteries.

Preferably, the invention relates to any of the compounds provided hereinabove for use in the prevention of the onset of or formation of or in reducing the risk of any of the following conditions (including one or more of the following conditions):
- thrombosis,
- coronary heart disease or coronary artery disease, including stenosis of the coronary arteries,
- myocardial infarction or heart attack,
- stroke, in particular ischemic stroke and/or hemorrhagic stroke,
- thrombosis, e.g. thrombosis formed over an atherosclerotic plaque,
- angina pectoris,
- peripheral arterial disease,
- aortic calcification, like calcific aortic valve disease (CAVD),
- atherosclerosis in renal dysfunctions like chronic kidney disease (CKD),
- atherosclerosis in neurodegenerative diseases, such as Alzheimer's disease.

Preferably, the invention relates to any of the compounds provided hereinabove for use in the treatment of or reversing the formation of any of the following conditions (including one or more of the following conditions):
- thrombosis,
- coronary heart disease or coronary artery disease, including stenosis of the coronary arteries,
- consequences of myocardial infarction or heart attack,
- stroke, in particular ischemic stroke and/or hemorrhagic stroke,
- thrombosis, e.g. thrombosis formed over an atherosclerotic plaque,
- angina pectoris,
- peripheral arterial disease,
- aortic calcification, like calcific aortic valve disease (CAVD),
- atherosclerosis in renal dysfunctions like chronic kidney disease (CKD),
- atherosclerosis in neurodegenerative diseases, such as Alzheimer's disease.

The invention also relates to any of the compounds provided hereinabove for use in reducing the amount of reactive oxygen species (ROS) and/or inhibiting ROS production; thereby reducing oxidative stress and preventing the onset of or alleviating oxidative stress diseases.

Preferably, in the therapeutic method, preferably preventive method, the compound is selected from the group consisting of compounds according to formula (I), wherein R₁ is C₇-C₁₃ alkyl; or any of the preferred compounds or any compound for use in treatment or prevention.

### Pharmaceutical compositions

The invention also relates to a pharmaceutical composition or medicament comprising the compound of the invention, wherein said compound is selected from the group consisting of compounds according to formula (I), wherein R₁ is C₇-C₁₃ alkyl; or any of the preferred compounds, and any pharmaceutically acceptable salt thereof, preferably its sodium salt,
as well as a pharmaceutically acceptable carrier or excipient.

Preferably the C₇-C₁₃ alkyl is n-alkyl, i.e. a linear alkyl.

Preferably, R₁ is C₉-C₁₃ alkyl.

Particularly preferably, R₁ is C₇-C₁₁ alkyl.

Particularly preferably, R₁ is C₁₁-C₁₃ alkyl.

In a further embodiment, R₁ is a C₈-C₁₂ or a C₉-C₁₂ alkyl.

In a further embodiment, R₁ is a C₈-C₁₁ or a C₉-C₁₁ alkyl.

In an embodiment, R₁ is C₁₀-C₁₂ alkyl.

More particularly preferably, R₁ is C₁₁ alkyl.

In a preferred embodiment, R₁ is any alkyl defined above. Preferably R₁ is n-alkyl.

Highly preferably, R₁ is C₁₁ n-alkyl, i.e. the compound is 17-lauroyloxy-5-androstene-3-sulphate.

Preferably the salt is sodium salt. Preferably the salt is potassium salt.

Preferably the alkyl is n-alkyl, i.e. a linear alkyl.

Preferably the salt is sodium salt. Preferably the solvate is water solvate.

In an embodiment the configuration at carbon 3 is 3β.

In an embodiment the configuration at carbon 17 is 17β.

In a preferred embodiment the configuration is 3β,17β.

In an embodiment the configuration at carbon 3 is 3α.

In an embodiment the configuration at carbon 17 is 17α.

The invention relates to a pharmaceutical composition comprising the compound according to the invention, said composition also comprising a pharmaceutically acceptable carrier or excipient. Preferably the compound according to the invention is a compound for use as defined herein; preferably a compound as defined above in the Compound section.

The invention relates to a pharmaceutical composition for use in a subject according to the invention for prevention against a condition as taught herein, said composition also comprising a pharmaceutically acceptable carrier or excipient.

Pharmaceutically acceptable carriers or excipients are known for a person skilled in the art.

Preferably the pharmaceutical composition is for or is suitable for enteral, e.g. oral or parenteral, e.g. intravenous, or topical administration into a patient. Oral administration is preferred.

Said oral pharmaceutical compositions may be formulated e.g. as pills, tablets, tabs, coated tablets, film tablets, capsules, powders, granulates, sustained-release formulations, suspensions or drops.

Intravenous pharmaceutical compositions may be formulated e.g. as liquid solution preparations for injections or infusion, and can be administered in any usual intravenous administration means, e.g. through an intravenous line (cannula) or by injections.

The pharmaceutical compositions may be formulated for topical administration, e.g. as drops, sprays, aerosols, ointments, creams, pastes, syrup, lotion or gels which may be useful for administration near to the site of the atherosclerotic lesion or plaque.

Thus, the pharmaceutical compositions may be formulated for systemic administration, like oral and intravenous compositions, or for local administration like topical compositions or other local administration compositions like sublingual, buccal, etc. administration.

Highly preferably the pharmaceutical composition is present in a form suitable for oral administration. The pharmaceutical composition in the form of tablets is most preferred.

In a preferred embodiment the pharmaceutical composition of the invention is formulated in a form for administration of a daily dose as defined below under Methods for treatment, preferably in one, two or three parts a day. Preferably the form is for oral administration.

In a preferred embodiment the administration form, in particular in human patients, comprises a dose of at least 0.5 mg, 1 mg, 2 mg, 5 mg, 10 mg, 15 mg, 20 mg or 30 mg.

In a preferred embodiment the administration form, in particular in human patients, comprises a dose of at most 500 mg, 200 mg, 150 mg or 100 mg, 80 mg, 50 mg or 40 mg.

In a preferred embodiment the administration form, in particular in human patients, comprises a dose between 0.5 and 500 mg, between 1 and 500 mg, or between 5 and 200 mg, or in particular between 10 and 100 mg, or between 20 and 200 mg, or between 5 and 100 mg, or in more particular between 10 and 80 mg, or 20 and 150 mg, or 1 to 30 mg or 1 to 40 mg or 1 to 60 mg, or 5 to 20 mg, or 5 to 40 mg or 5 to 60 mg or 10 to 40 mg.

In another preferred embodiment, the administration dose for a human is 10 to 40 mg/day.

In another preferred embodiment, the administration dose for a human is between 0.01 mg/kg and 5 mg/kg or between 0.01 mg/kg and 1 mg/kg, preferably between 0.01 mg/kg and 0.5 mg/kg, in particular between 0.01 mg/kg and 0.4 mg/kg, or between 0.01 mg/kg and 0.3 mg/kg or between 0.01 mg/kg and 0.05 mg/kg, or between 0.05 mg/kg and 0.5 mg/kg. In these doses, per kg refers to per kilogram of body weight.

In another preferred embodiment, the administration dose for a mouse is a dose between 1 µg/day and 200 µg/day, in particular 4 µg/day to 100 µg/day or 4 µg/day to 20 µg/day or 20 µg/day to 100 µg/day.

In another preferred embodiment, the administration dose for a mouse is a dose between 0.1 mg/kg and 10 mg/kg, in particular between 0.1 mg/kg and 5 mg/kg, or between 0.1 mg/kg and 1 mg/kg, or between 0.5 mg/kg and 5 mg/kg or between 1 mg/kg and 5 mg/kg. In these doses, per kg refers to per kilogram of body weight.

In an embodiment, the dose is a dose corresponding to any of the above doses calculated for another animal under appropriate guidelines (see e.g. U.S. Department of Health and Human Services, Food and Drug Administration, Center for Drug Evaluation and Research (2005). Guidance for Industry - Estimating the Maximum Safe Starting Dose in Initial Clinical Trials for Therapeutics in Adult Healthy Volunteers).

### Medicinal indications

The references to methods of treatment in the summary and detailed description of the invention in this description are to be interpreted as references to the compounds, pharmaceutical compositions and medicaments of the present invention for use in a method for treatment of the human (or animal) body by therapy (or for diagnosis).

The invention also relates to methods for treatment, wherein the pharmaceutical composition is administered to a subject or patient in an effective dose.

According to the invention the administration routes listed above in connection with the pharmaceutical compositions can be used in the methods for treatment of the invention for administration of the composition to a subject. The subject is a vertebrate having a blood circulation system (cardiovascular system or the vascular system), e.g. a mammalian or human subject as defined herein.

In a preferred embodiment the daily dose of administration, in particular in human patients, is at least 0.5 mg, 1 mg/day, 5 mg/day, 10 mg/day, 20 mg/day or 30 mg/day.

In a preferred embodiment the daily dose of administration, in particular in human patients, is at most 500 mg/day, 200 mg/day, 150 mg/day or 100 mg/day or 80 mg/day.

In a preferred embodiment the daily dose of administration, in particular in human patients, is between 0.5 and 500 mg/day, between 1 and 500 mg/day, or between 5 and 200 mg/day, or in particular between 10 and 100 mg/day, or between 20 and 200 mg/day, or between 5 and 100 mg/day, or in more particular between 10 and 80 mg/day, or 20 and 150 mg/day, or 1 to 30 mg or 1 to 40 mg or 1 to 60 mg, or 5 to 20 mg, or 5 to 40 mg or 5 to 60 mg or 10 to 40 mg.

In another preferred embodiment, the administration dose for a human is 10 to 40 mg/day.

In another preferred embodiment, the administration dose for a human is between 0.01 mg/kg and 5 mg/kg or between 0.01 mg/kg and 1 mg/kg, preferably between 0.01 mg/kg and 0.5 mg/kg, in particular between 0.01 mg/kg and 0.4 mg/kg, or between 0.01 mg/kg and 0.3 mg/kg or between 0.01 mg/kg and 0.05 mg/kg, or between 0.05 mg/kg and 0.5 mg/kg. In these doses, per kg refers to per kilogram of body weight.

In another preferred embodiment, the administration dose for a mouse is a dose between 1 µg/day and 200 µg/day, in particular 4 µg/day to 100 µg/day or 4 µg/day to 20 µg/day or 20 µg/day to 100 µg/day.

In another preferred embodiment, the administration dose for a mouse is a dose between 0.1 mg/kg and 10 mg/kg, in particular between 0.1 mg/kg and 5 mg/kg, or between 0.1 mg/kg and 1 mg/kg, or between 0.5 mg/kg and 5 mg/kg or between 1 mg/kg and 5 mg/kg. In these doses, per kg refers to per kilogram of body weight.

In an embodiment, the dose is a dose corresponding to any of the above doses calculated for another animal under appropriate guidelines (see e.g. U.S. Department of Health and Human Services, Food and Drug Administration, Center for Drug Evaluation and Research (2005). Guidance for Industry - Estimating the Maximum Safe Starting Dose in Initial Clinical Trials for Therapeutics in Adult Healthy Volunteers).

In an embodiment the effective dose means the administration of an amount, wherein the level of H₂S is detectably or effectively elevated in the subject or patient.

The invention relates to a method for the treatment of atherosclerosis.

In the method of the invention, the compound of the invention is administered as disclosed herein to a subject, e.g. to a mammalian subject, preferably a human subject.

In an embodiment the method for treatment is a method for prevention or reducing the risk of atherosclerosis, or for prevention of or for reducing the formation of or for alleviating the formation of or for reducing the risk of atherosclerotic lesions or plaques in arteries.

In an embodiment the method for treatment is a method for the reversion of atherosclerosis or of the formation of atherosclerotic lesions or plaques in arteries.

Preferably, the invention relates to the method of treatment as taught herein, wherein said compound or composition is administered in the prevention of onset or formation of or in reducing the risk of any of the conditions listed above in the Compounds section or
to the method of treatment as taught herein, wherein said compound or composition is administered in the treatment of or reversing the formation of any of the conditions listed above in the Compounds section.

In an embodiment the mammalian or human subject is diagnosed with atherosclerosis before said method of treatment is started.

In an embodiment the mammalian or human subject is diagnosed as a subject at risk of atherosclerosis before said method of treatment is started.

Such diagnostic methods are well known to a person skilled in the art as taught hereinbelow.

### Process for the preparation

The invention also relates to a process for the preparation of a compound of the invention, said method comprising the steps of
I. conversion of 3-hydroxy-17-oxo-5-androstene to obtain 3-acetoxy-17-oxo-5-androstene by acylation,
II. conversion of the 3-acetoxy-17-oxo-5-androstene to 3-acetoxy-17-hydroxy-5-androstene by reduction,
III. conversion of the 3-acetoxy-17-hydroxy-5-androstene to 3-acetoxy-17-C₈-C₁₄-alkanoyloxy-5-androstene by acylation, preferably with a C₈₋₁₄-alkanoyl halogenide, preferably C₈₋₁₄-alkanoyl chloride.
IV. conversion of the 3-acetoxy-17-C₈-C₁₄-alkanoyloxy-5-androstene to 3-hydroxy-17-C₈-C₁₄-alkanoyloxy-5-androstene by hydrolysis,
V. conversion of the 3-hydroxy-17-C₈-C₁₄-alkanoyloxy-5-androstene to 17-C₈-C₁₄-alkanoyloxy-5-androstene-3-sulphate by sulphation,
VI. optionally converting the 17-C₈-C₁₄-alkanoyloxy-5-androstene-3-sulphate to a salt, in particular a pharmaceutically acceptable salt thereof, preferably sodium salt.

In a preferred embodiment R₁ is C₇-C₁₃ alkyl. Particularly preferably, R₁ is C₇-C₁₁ alkyl. Particularly preferably, R₁ is C₁₁-C₁₃ alkyl.

Preferably R₁ is n-alkyl.

In another preferred embodiment, R₁ is C₉-C₁₃ alkyl.

In a further embodiment, R₁ is a C₈-C₁₂ or a C₉-C₁₂ alkyl.

In a further embodiment, R₁ is a C₈-C₁₁ or a C₉-C₁₁ alkyl.

In an embodiment, R₁ is C₁₀-C₁₂ alkyl.

More particularly preferably, R₁ is C₁₁ alkyl.

In a preferred embodiment, R₁ is any alkyl defined above. Preferably R₁ is n-alkyl.

In a preferred embodiment, in acylation step III a C₁₀-C₁₄-alkanoyloxy-halogenide is used and thereby in step V of the process 17-C₁₀-C₁₄-alkanoyloxy-5-androstene-3-sulphate is obtained.

In a particularly preferred embodiment, in acylation step III a C₈-C₁₂-alkanoyloxy-halogenide is used and thereby in step V of the process 17-C₈-C₁₂-alkanoyloxy-5-androstene-3-sulphate is obtained.

In a particularly preferred embodiment, in acylation step III a C₁₂-C₁₄-alkanoyloxy-halogenide is used and thereby in step V of the process 17-C₁₂-C₁₄-alkanoyloxy-5-androstene-3-sulphate is obtained.

In a highly preferred embodiment, in acylation step III a C₁₂-alkanoyloxy-halogenide is used and thereby in step V of the process 17-C₁₂-alkanoyloxy-5-androstene-3-sulphate is obtained.

In an embodiment acylation step I is carried out by reacting the 3-hydroxy-17-oxo-5-androstene with acetic anhydride under appropriate conditions, e.g. in an aprotic apolar solvent, e.g. in dichloromethane, preferably at temperature T = 0°C, preferably for 2 to 4 hours.

In an embodiment reduction step II is carried out by reacting the 3-acetoxy-17-oxo-5-androstene with a reducing agent, e.g. a hydride, in particular NaBH₄, under appropriate conditions, e.g. in a protic polar solvent, e.g. in methanol, preferably at temperature T < 0°C, more preferably at -10°C < T < 0°C, even more preferably at -8°C < T < -2°C, preferably for 1 to 5 hours, preferably for 2 to 3 hours.

In an embodiment acylation step III is carried out by reacting the 3-acetoxy-17-hydroxy-5-androstene with the respective C₈-C₁₄-alkanoyloxy-halogenide, preferably C₈-C₁₄-alkanoyloxy-chloride, under appropriate conditions, e.g. in an aprotic solvent, preferably in a mixture of an apolar and an aromatic solvent, preferably in a mixture of dichloromethane and pyridine, preferably at room temperature or at a temperature of 20°C < T < 30°C, preferably for less than one day, or 2 to 24 hours, preferably 4 to 16 hours.

In an embodiment hydrolysis step IV is carried out by hydrolyzing the 3-acetoxy-17-C₈-C₁₄-alkanoyloxy-5-androstene under basic conditions, preferably by using an alkali hydroxide, e.g. sodium hydroxide in a protic polar solvent, e.g. in a mixture of methanol and water, at a temperature of 10°C < T < 30°C, preferably at a temperature of 15°C < T < 25°C preferably for less than one day, or 4 to 24 hours, preferably 6 to 20 hours.

In an embodiment sulphation step V is carried out by sulphating the 3-hydroxy-17-C₈-C₁₄-alkanoyloxy-5-androstene with a sulphating agent, preferably chlorosulfonic acid, preferably in a mixture of an apolar and an aromatic solvent, preferably in a mixture of dichloromethane and pyridine, preferably at a temperature of T < 0°C, more preferably at -10°C < T < 0°C, even more preferably at -8°C < T < -2°C, preferably for 1 to 12 hours, preferably for 2 to 8 hours.

In a preferred embodiment appropriate salt is formed by using a metal salt forming reagent, preferably an alkali metal salt, e.g. in water comprising preferably the corresponding alkali carbonate or hydrogen carbonate, preferably by adding the metal salt forming reagent to the sulphating reaction mixture once the sulphating reaction is allowed to proceed for a sufficient time. Optionally salt is formed in a multiple phase, e.g. three-phase reaction, which is diluted by an apolar volatile solvent, e.g. diethyl ether and the salt is filtered out and purified, optionally recrystallized.

In a preferred embodiment the process of the invention comprises the steps of
- converting 3-hydroxy-17-oxo-5-androstene (10) (DHEA) into 3-acetoxy-17-oxo-5-androstene (1) by acylation,
- converting 3-acetoxy-17-oxo-5-androstene (1) into 3-acetoxy-17-hydroxy-5-androstene (2) by reduction,
- converting 3-acetoxy-17-hydroxy-5-androstene (2) into 3-acetoxy-17-lauroyloxy-5-androstene (3) by acylation,
- converting 3-acetoxy-17-lauroyloxy-5-androstene (3) into 3-hydroxy-17-lauroyloxy-5-androstene (4) by hydrolysis,
- converting 3-hydroxy-17-lauroyloxy-5-androstene (4) into 17-lauroyloxy-5-androstene-3-sulphate by sulphation, preferably into 17-lauroyloxy-5-androstene-3-sulphate sodium salt (5).

### ABBREVIATIONS

- ApoE^{-/-}: apolipoprotein E-deficient mice
- C6: 17-hexanoyloxy-5-androstene-3-sulfate sodium salt
- C16: 17-palmitoyloxy-5-androstene-3-sulfate sodium salt
- CAD (also CHD): coronary artery disease
- CAVD: calcific aortic valve disease
- CHD (also CAD): coronary heart disease
- CKD: chronic kidney disease
- DCM: dichloromethane
- DHEA: Dehydroepiandrosterone
- DHEA-S or DHEAS: Dehydroepiandrosterone sulfate
- H₂S: Hydrogen sulfide
- LEAD: lower extremity arterial disease
- MB: methylene blue
- PAD: peripheral arterial disease
- ROS: reactive oxygen species
- S2: 17-lauroyloxy-5-androstene-3-sulphate sodium salt (DHEA-derived steroid or Steroid 2)
- TSC: total scavenger capacity

### DEFINITIONS

"Arteriosclerosis" is a condition wherein the walls of arteries are thickened, hardened, and lost their elasticity. Arteriosclerosis is considered herein as a disease.

"Atherosclerosis" is a specific type of arteriosclerosis wherein abnormal material is accumulated in the inner layer of the wall of an artery, which forms "atherosclerotic lesions", which are made up of fats, cholesterol and other substances in and on the walls of arteries and restrict blood flow.

"Atherosclerotic lesions" are abnormalities in the inner side of the arterial wall and have several types (types I to VI) based on their size, composition and severity. Less developed atherosclerotic lesions do not cause symptoms.

"Atherosclerotic plaques" or atheromas are abnormal accumulation of material in the inner layer of the wall of an artery, in particular atherosclerotic lesions which comprise extracellular lipids which are confluent, and which potentially cause symptoms in the subject affected by atherosclerosis.

A "subject" as used herein is an individual of an animal species having blood circulation and capable of developing arteriosclerosis or atherosclerosis, preferably a vertebrate, more preferably a mammalian or avian species, in particular a mammalian species, highly preferably the individual is a primate, a hominid or a human.

A "patient" is a subject who is or intended to be under medical or veterinarian observation, supervision, diagnosis or treatment.

A "treatment" refers to a process or method of administration of a pharmaceutical composition or medicament to a subject (or patient), wherein the subject or patient is under medical or veterinarian aid with the object of improving the subject's or patient's condition or restoring his/her health, either directly or indirectly or maintaining it in a healthy or stabilized state to avoid worsening. Treatment of the subject as used herein include restoring or maintaining normal function of an organ or tissue, preferably at least partly restoring or maintaining health (medical or veterinarian treatment). Treatment typically refers to the administration of an effective amount of a compound or composition described herein. Treatment may relate to or include medical or veterinarian treatment and cosmetic treatment, in particular medical or veterinarian treatment.

A "therapeutic treatment" (or therapy) is a treatment with the object of improving the subject's or patient's condition or restoring his/her health. For example, as used herein, the therapy includes reversion of atherosclerosis or reversion of the formation of atherosclerotic lesions in arteries.

A "preventive treatment" (or prevention) is a treatment with the object of preventing or avoiding the onset of or worsening of a disease or condition. For example, as used herein, prevention includes reducing the formation of or alleviating the formation of or reducing the risk of atherosclerotic lesions in arteries.

A "pharmaceutical composition" or "composition" of the invention is a composition of matter which comprises at least one biologically active substance of the invention suitable for increasing H₂S level in the subject and thereby preventing the formation of atherosclerotic lesions or plaques. The treatment includes administration of the substance in an effective amount. Compositions may also comprise further biologically active substances useful e.g. in a combination therapy. Furthermore, the compositions may comprise pharmaceutically acceptable carriers and/or excipients, including formulation agents, etc., which are well known in the art. A medicament is a "pharmaceutical composition" marketed or to be marketed under a marketing authorization issued or to be issued by a respective authority in the geographic area given.

The terms "effective amount" and "therapeutically effective amount" are intended to qualify the amount of a therapeutic agent required to relieve or prevent to some extent one or more of the symptoms of a condition.

As used herein, the term "alkyl" alone or in combinations means a straight or branched-chain saturated hydro-carbon group of a length as given by the number of carbon atom. In a broader sense the alkyl may comprise substituents, e.g. halogen substituents. In a narrower sense the alkyl does not comprise a substituent.

As used herein, the term "alkanoyl" refers to a group which contains a double-bonded oxygen atom and an alkyl group having the formula R-C=O or R-(CO)-, wherein R represents an alkyl group that is linked to the carbon atom of the group by a single bond and the carbon atom may be linked by a single covalent bond to another moiety of the organic molecule. In organic chemistry, the alkanoyl group is usually derived from a carboxylic acid.

When used herein, the terms "halogen" and "halo" include fluorine, chlorine, bromine and iodine, and fluoro, chloro, bromo and iodo, respectively.

"Sulphation" (or "sulfation") means herein a process wherein a sulphate ester is formed in an organic molecule.

As used herein, the term "solvate" is a crystal form containing the compound of the invention or a pharmaceutically acceptable salt thereof and either a stoichiometric or a non-stoichiometric amount of a solvent, by way of example, water. Reference to a compound of the invention includes any physical form of that compound, unless a particular form, salt or solvate thereof is specified.

The term "comprises" or "comprising" or "including" are to be construed herein as having a non-exhaustive meaning and allow the addition or involvement of further features or method steps or components to anything which comprises the listed features or method steps or components. "Comprising" can be substituted by "including" if the practice of a given language variant so requires or can be limited to "consisting essentially of" if other members or components are not essential to reduce the invention to practice.

The indefinite articles "a" or "an" may refer either to singular or plural if context allows.

### BRIEF DESCRIPTION OF THE FIGURES

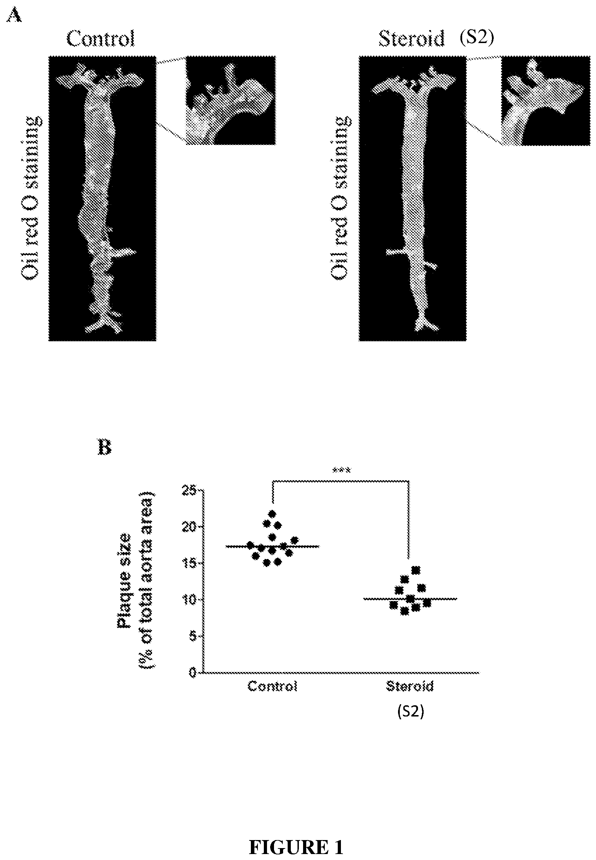

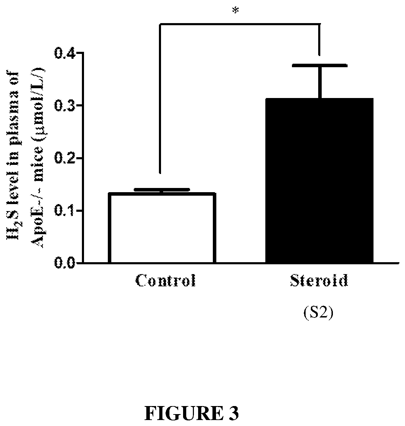

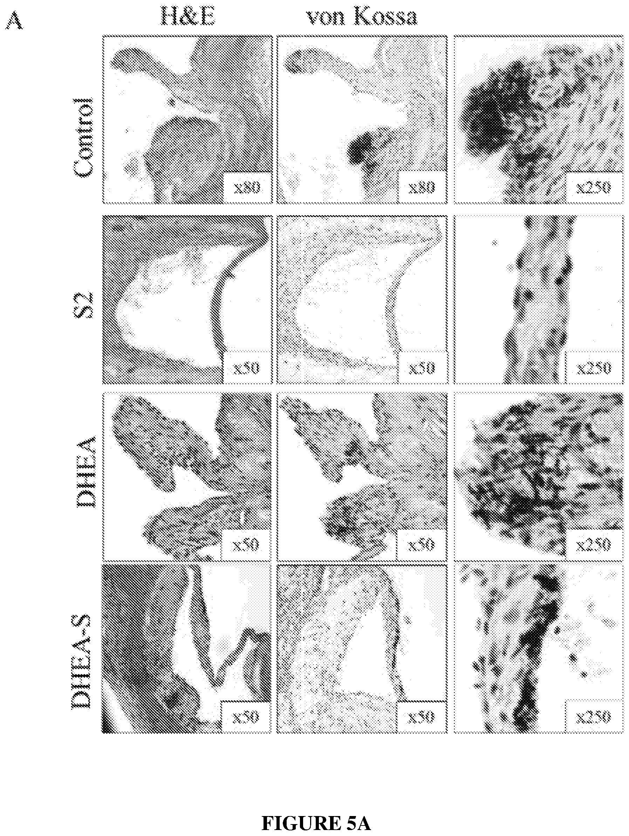

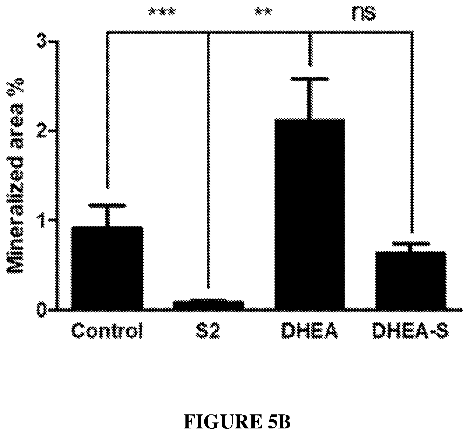

***Figure 1******. DHEA-derived steroid inhibits atherosclerosis***
   (A) Representative Oil Red O staining of aortas dissected from ApoE^{-/-} mice fed with atherogenic diet and treated with DHEA-derived steroid (N=9) or control (tap water, N=13) for 8 weeks were already demonstrated. (B) Quantification of atherosclerotic plaque size was performed with Image J software. The graph shows the mean ±SEM of tap-water (N=13) and DHEA-derived steroid (N=9) mice aorta. The differences in means were analyzed by Student's t-test. ***P < 0.001.

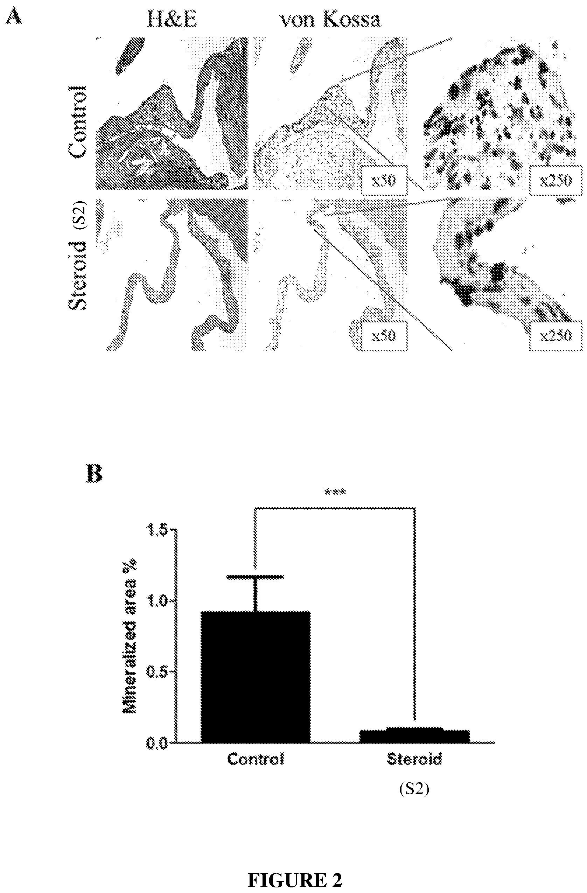

***Figure 2******. DHEA-derived steroid decreases the expansion of the extracellular matrix***
   (A) Hematoxylin and eosin (left panels, x400 magnification); von Kossa (middle and right panels) staining was performed on aortic valves of mice kept on a high-fat diet and tap-water (upper panels; N=13), and on high-fat diet treated with DHEA-derived steroid (lower panels; N=9). Immunohistochemistry of von Kossa was shown with two different magnifications (x400 magnification and x1000 magnification). (B) Quantification of the mineralized area of the mice heart valves was performed with Image J software. The graph shows the mean ±SEM of tap-water (N=13) and DHEA-derived steroid (N=9) mice aorta. The differences in means were analyzed by Student's t-test. ***P < 0.001
***Figure 3******. DHEA-derived steroid elevates H₂S content of the mice plasma***
   Zn²⁺ precipitated sulfide content under alkaline conditions was measured in tap-water (N=4) and DHEA-derived steroid (N=4) treated mice plasma normalized to the protein content of the samples were shown. The graph shows the mean ±SEM of tap-water (N=4) and DHEA-derived steroid (N=4) mice plasma H₂S contents. The differences in means were analyzed by Student's t-test. *P < 0.05.

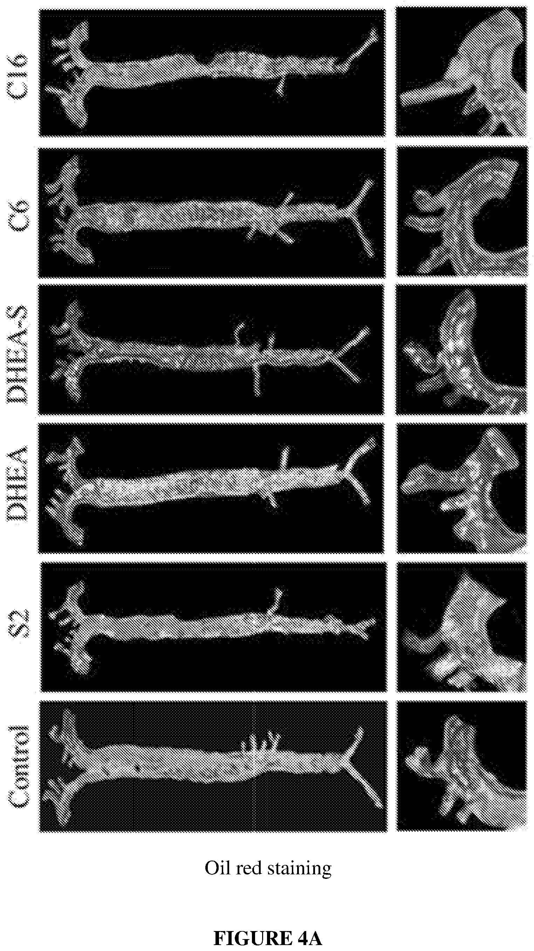

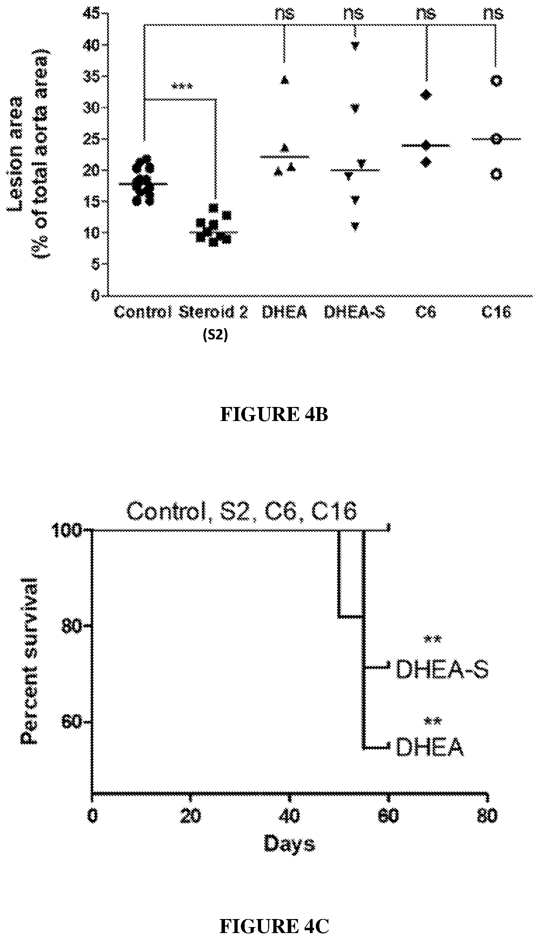

***Figure 4******. DHEA, DHEA-S, C6 and C16fail to inhibit atherosclerosis***
   (A) Representative Oil Red O staining of aortas dissected from ApoE^{-/-} mice fed with atherogenic diet and treated with DHEA (N=11), DHEA-S (N=7), C6 (N=3) and C16 (N=3) for 8 weeks were already demonstrated. (B) Quantification of atherosclerotic plaque size was performed with Image J software. The graph shows the mean ±SEM of control (N=13), S2 (N=9), DHEA (N=5), DHEA-S (N=6), C6 (N=3) and C16 (N=3) mice aorta. The differences in means were analyzed by 1-way ANOVA with Dunett post-test. ***P < 0.001; ns: non-significant. (C) Survival of control, S2, DHEA, DHEA-S, C6 and C16 mouse groups were shown. Mice in the control group as well as mice treated with S2, C6 and C16 survived (did not die) during the experiment. However, 45% of mice treated with DHEA and 22% of mice treated with DHEA-S died before the end of the experiment. Mice treated with DHEA started to die earlier during the treatment than mice treated with DHEA-S. The first DHEA treated animal died on day 50 whereas the first DHEA-S treated animal died on day 55 of the treatment regime (i.e. start of the treatment with S2, DHEA, DHEA-S, C6 or C16).
   The comparison of survival curves was analyzed with Mantel-Cox test. **P < 0.01
***Figure 5******. DHEA and DHEA-S cannot decrease the mineralization of extracellular matrix of aortic valve of ApoE knock out mice***
   (A) Hematoxylin and eosin (left panels, x50 magnification); von Kossa (middle (x50 magnification) and right panels (x250 magnification)) staining was performed on aortic valves of mice kept on a high-fat diet and tap-water (first row; N=13), on high-fat diet treated with DHEA-derived S2 steroid (second row; N=9), on high-fat diet treated with DHEA (third row; N=11), and on high-fat diet treated with DHEA-S (fourth row; N=7). (B) Quantification of the mineralized area of the mice heart valves was performed with Image J software. The graph shows the mean ±SEM of tap-water (N=13), DHEA-derived steroid (N=9), DHEA (N=8) and DHEA-S (N=9) mice aorta. The differences in means were analyzed by Student's t-test. ***P < 0.001; **P < 0.01; ns: non-significant.

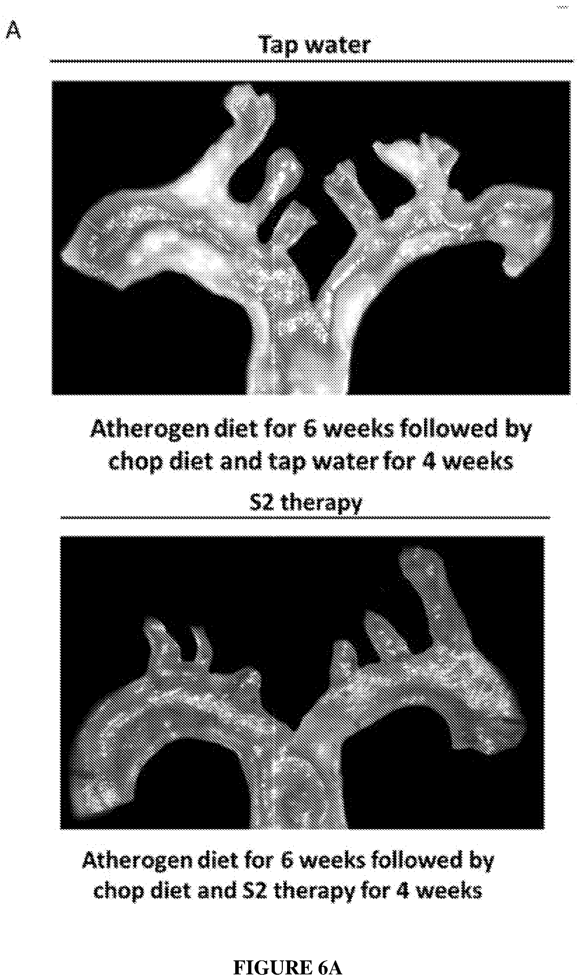

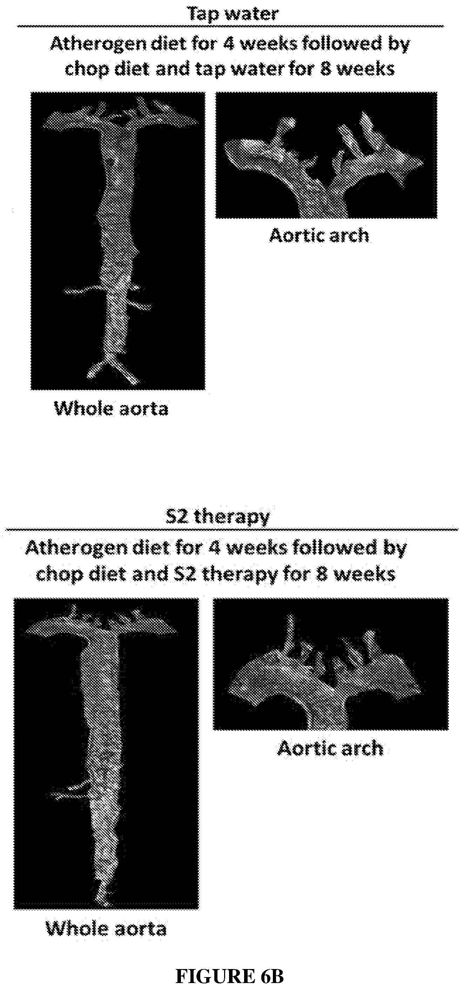

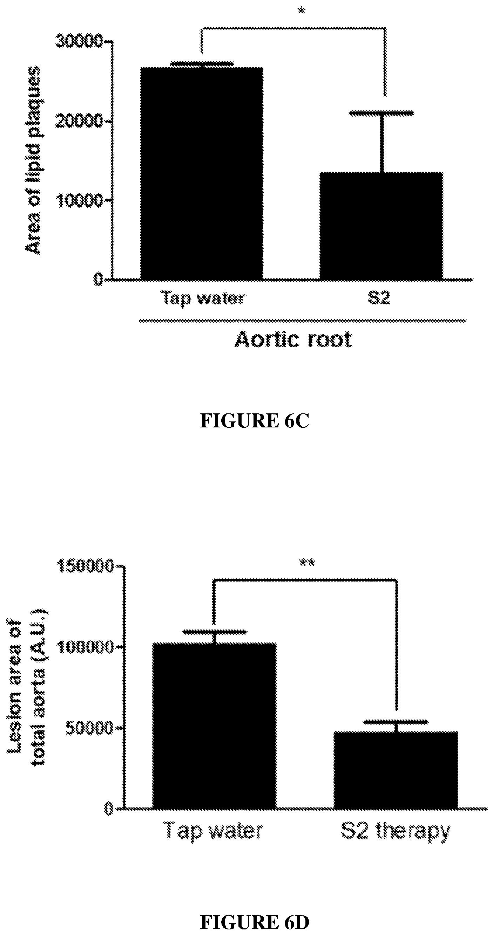

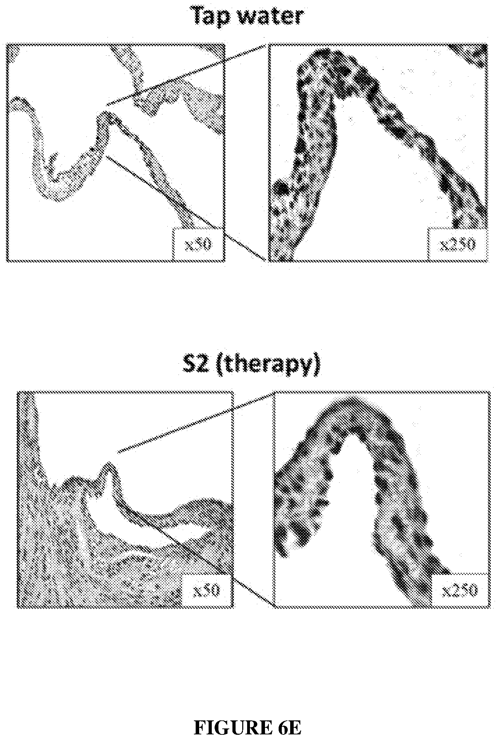

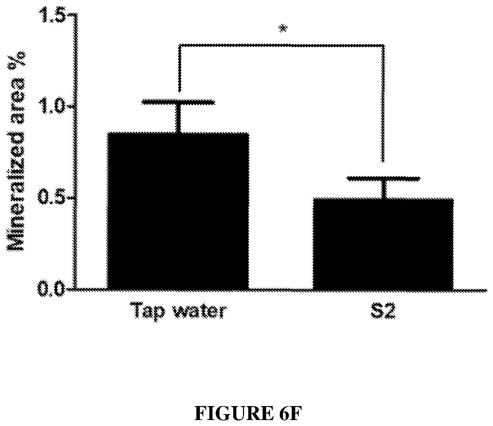

***Figure 6******. DHEA-derived S2 steroid reverses atherosclerosis and aortic valve calcification***
   (A) Representative Oil Red O staining of aortas dissected from ApoE^{-/-} mice fed with atherogenic diet for 6 weeks followed by chop diet and treated with DHEA-derived S2 steroid (N=3) or control (tap water, N=3) for 4 weeks were already demonstrated.
   (B) Representative Oil Red O staining of aortas dissected from ApoE^{-/-} mice fed with atherogenic diet for 4 weeks followed by chop diet and treated with DHEA-derived S2 steroid (N=5) or control (tap water, N=3) for 8 weeks were already demonstrated.
   (C) Quantification of atherosclerotic plaque size measured in the aortas shown on panel A was performed with Image J software. The graph shows the mean ±SEM of tap-water (N=3) and DHEA-derived steroid (N=3) mice aorta. The differences in means were analyzed by Student's t-test. *P < 0.05.
   (D) Quantification of plaque size in experiments shown on panel B was performed with Image J software. The graph shows the mean ±SEM of tap-water (N=3) and DHEA-derived steroid (N=5) mice aorta. The differences in means were analyzed by Student's t-test. **P < 0.01.
   (E) Von Kossa (x50 magnification and x250 magnification) staining was performed on aortic valves of mice kept on a high-fat diet for 6 weeks followed by chop diet and tap water (N=3) or S2 steroid therapy (N=3) in tap water for 4 weeks.
   (F) Quantification of the mineralized area of the mice heart valves shown on panel E was performed with Image J software. The graph shows the mean ±SEM of tap-water (N=3) and S2 steroid (N=3) mice aorta. The differences in means were analyzed by Student's t-test. *P < 0.05.

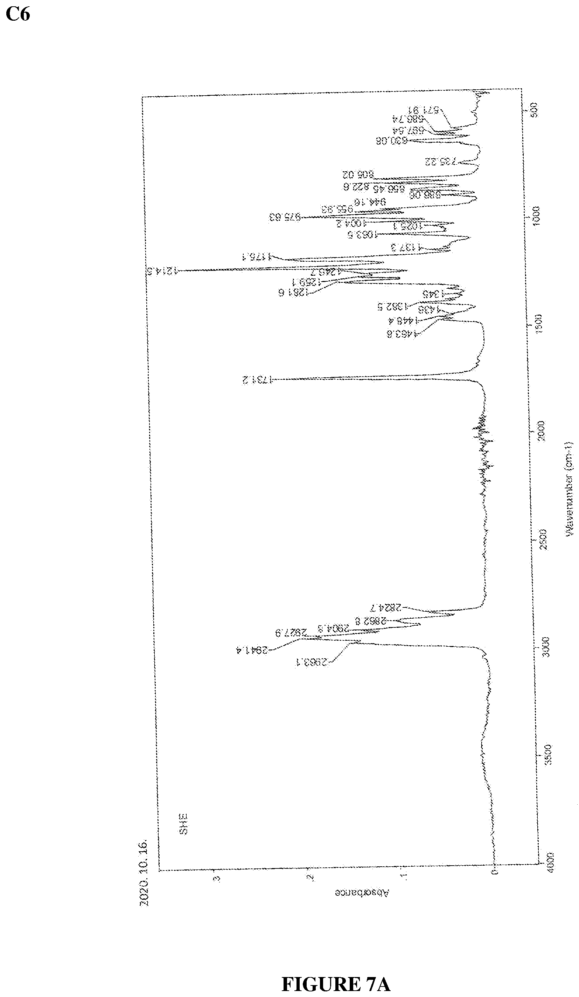

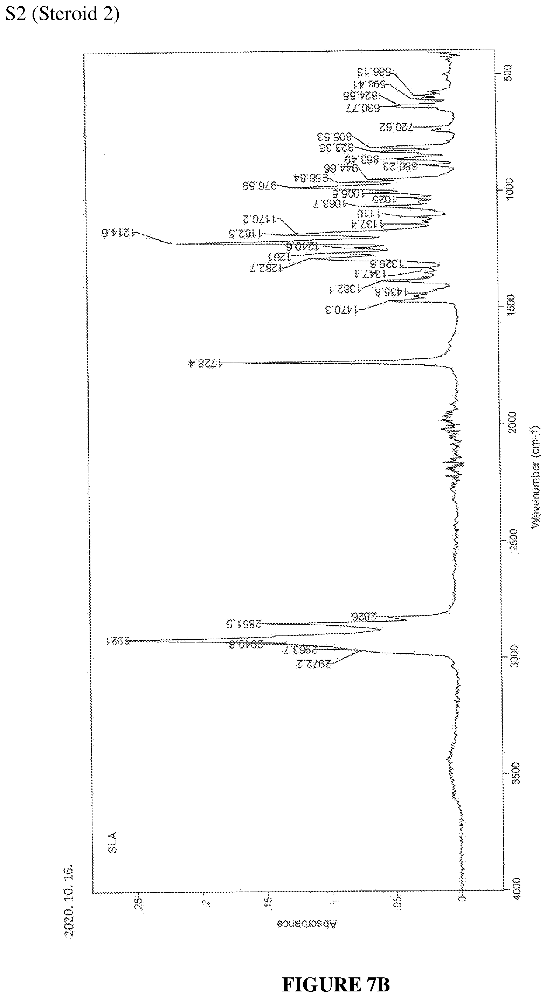

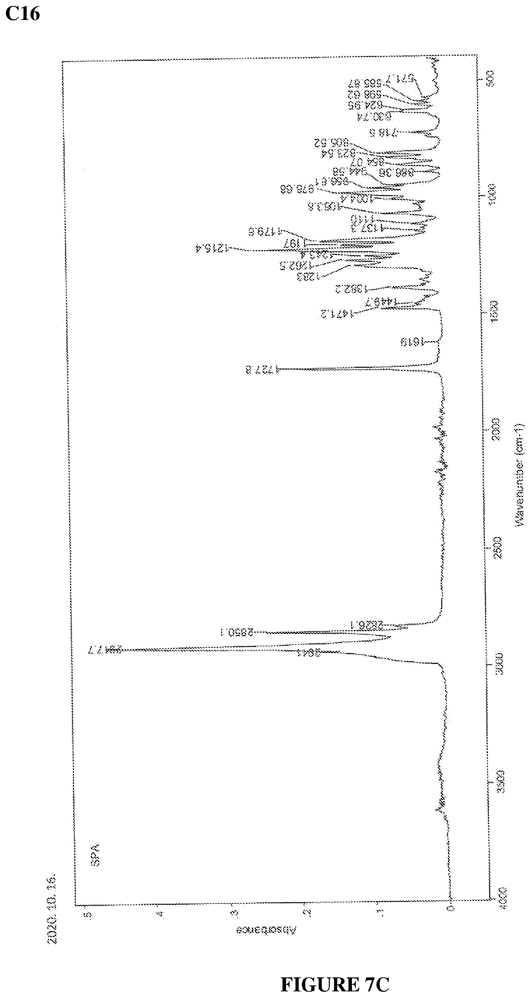

***Figure 7******. NMR records of the compounds of the invention***
   (A) NMR record of C6 steroid (17-hexanoyloxy-5-androstene-3-sulphate sodium salt)
   (B) NMR record of S2 steroid (17-lauroyloxy-5-androstene-3-sulphate sodium salt)
   (C) NMR record of C16 steroid (17-palmitoyloxy-5-androstene-3-sulphate sodium salt)

### DETAILED DESCRIPTION OF THE INVENTION

Arteriosclerosis and atherosclerosis are diseases wherein the walls of arteries are thickened, hardened, and lost their elasticity, and in atherosclerosis blood flow may be hindered. These conditions may lead to more serious diseases. The causes of arteriosclerosis and atherosclerosis appear to be lipid retention and modification, oxidation by free radicals (oxidative stress), which provoke chronic inflammation at susceptible sites in the walls. Initial lesions including fatty streaks in arteriosclerosis evolve into fibrous atherosclerotic plaques, which may become vulnerable to rupture, and thus may cause thrombosis or stenosis [Insull, W 2009]. Risk factors for atherosclerosis and its thrombotic complications include hypertension, cigarette smoking and diabetes mellitus, and the role of the immune system is inevitable, chronic inflammation being a risk factor in itself.

Type I lesion contains atherogenic lipoprotein which elicit an increase in macrophages and formation of scattered macrophage foam cells. Type II lesion, also called as fatty streaks are formed by layers of macrophage foam cells and smooth muscle cells carrying lipids. Type III lesions contain scattered extracellular lipid droplets and particles that disrupt the coherence of some intimal smooth muscle cells. Type IV lesions also comprise extracellular lipids which are confluent and larger than in Type III lesions (the latter producing a transitional state between type II and type IV lesions). A type IV lesion and more serious lesions are also called atherosclerotic plaques or atheromas and are already potentially symptom-producing. Type V lesions usually have a lipid core and also contain layer(s) of fibrous connective tissue, whereas type VI lesions comprise fissure, hematoma, and thrombus (type VI lesion). Type V lesions have variants, and e.g. type Vb lesions are largely calcified and type Vc lesions consist mainly of fibrous connective tissue and little or no accumulated lipid or calcium. The plaque can burst, triggering a blood clot [Stary H C. *et al.* 1995].

The role of free radicals is investigated in connection with arteriosclerosis and atherosclerosis.

Free radicals are molecules that contain an unpaired electron on their outer atomic orbital. These seek a pair for themselves, and accept an electron from their surroundings, thus a chain of production of new molecules with unpaired electrons begins. Free radicals are therefore highly reactive and are able to damage the surrounding biological substances, like proteins, carbohydrates, lipids, and nucleic acids. This leads to the development of free radical mediated diseases, like atherosclerosis.

Earlier research with the radical neutralizing, antioxidant, also known as scavenging effect of steroid hormones, have proven that a number of steroids, like sex hormones and cortisol are effective antioxidants in isolated human neutrophil granulocytes. It has also been shown in the art that these steroids, besides the antioxidant effect, also have strong endocrinological effect, thus their preventive administration in human medicine is out of the question.

The present inventors have started from a very weak androgen, dehydroepiandrosterone (DHEA) as initial molecule and surprisingly found that the binding of certain substituents to the 3rd and 17th carbon atom of the sterane frame bears antioxidant property. Besides preventing the formation of atherosclerotic lesions and plaques in an animal model, the DHEA-derived steroids according to the invention reverse the atherosclerotic lesions and plaques previously formed in the animals.

Thus, the DHEA-derived steroids of the invention represent a novel atheroprotective drug, usable for preventing the formation of lipid derivatives in the atherosclerotic lesions and plaques, and inhibiting and also reversing calcification of heart valves.

Using organic chemical methods, the present inventors placed a sulfate group in the 3rd position and a lauroyloxy hydrocarbon chain in the 17th position. Therefore, the present inventors created a set of novel steroid compounds, 17-alkyloxy-5-androstene-3-sulphates, which proved to be highly antioxidant in isolated human neutrophil granulocytes, without substantive endocrinological effect. In a preferred embodiment the 17 alkyl is n-alkyl.

The chemical name of a highly preferred new compound is: 17-lauroyloxy-5-androstene-3-sulphate (or 5-androstenediol-17-lauroyl-3-sulphate).

In particular embodiments the compound is selected from the group consisting of 17β-lauroyloxy-5-androstene-3β-sulphate, 17β-lauroyloxy-5-androstene-3α-sulphate, 17α-lauroyloxy-5-androstene-3β-sulphate and 17α-lauroyloxy-5-androstene-3α-sulphate. In a preferred embodiment the compound is 17β-lauroyloxy-5-androstene-3β-sulphate.

In a particular embodiment its sodium salt is used. The chemical formula is C₃₁H₅₁NaO₆S.

The chemical steps were the following: 1: DHEA, 2: 3-acetoxy-17-oxo-5-androstene, 3: 3-acetoxy-17-hydroxy-5-androstene, 4: 3-acetoxy-17-lauroyloxy-5-androstene, 5: 3-hydroxy-17-lauroyloxy-5-androstene, 6: 17-lauroyloxy-5-androstene-3-sulphate sodium salt. See below in detail.

A preferred compound has a hydrocarbon chain with 12 carbon atoms in the 17th position. We examined whether compounds with shorter (6 carbon atoms: 17-hexanoyloxy-5-androstene-3-sulphate sodium salt, see compound C6) or longer (16 carbon atoms: 17-palmitoyloxy-5-androstene-3-sulphate sodium salt, see compound C16) hydrocarbon chain have antiatherogenic effect. In animals treated with these compounds the size and the number of plaques were similar to the control mice (while a very small non-significant effect may have been present in case of compound C16). However, 17-lauroyloxy-5-androstene-3-sulphate sodium salt (see compound S2) proved to be highly efficient both in the prevention and reversal of atherosclerosis. This means, that the positive effect of the newly synthesized compound of the invention is bound to the range between 6 and 16 carbon atoms. Thus, the length of the chain of the alkanoyl group is at least 8 carbon atoms, i.e. R₁ is C7 in general formula (I), i.e. n is 6 in general formula (I') and (V), and at most 14 carbon atoms, i.e. R₁ is C13 in general formula (I), i.e. n is 12 in general formula (I') and (V).

Preferably, the length of the chain of the alkanoyl group is at least 10 carbon atoms, i.e. R₁ is C9 in general formula (I), i.e. n is 8 in general formula (I') and (V), and at most 14 carbon atoms, i.e. R₁ is C13 in general formula (I), i.e. n is 12 in general formula (I') and (V).

In a particular embodiment, the length of the chain of the alkanoyl group is at least 11 carbon atoms, i.e. R₁ is C10 in general formula (I), i.e. n is 9 in general formula (I') and (V), and at most 13 carbon atoms, i.e. R₁ is C12 in general formula (I), i.e. n is 11 in general formula (I') and (V).

In a highly preferred embodiment, the length of the chain of the alkanoyl group is 12 carbon atoms, i.e. R₁ is C₁₁ in general formula (I), i.e. n is 10 in general formula (I') and (V).

The exemplary compound of the invention used in the experiments below is a 17-lauroyloxy-5-androstene-3-sulphate salt, preferably sodium salt.

The compound has the general formula (I') 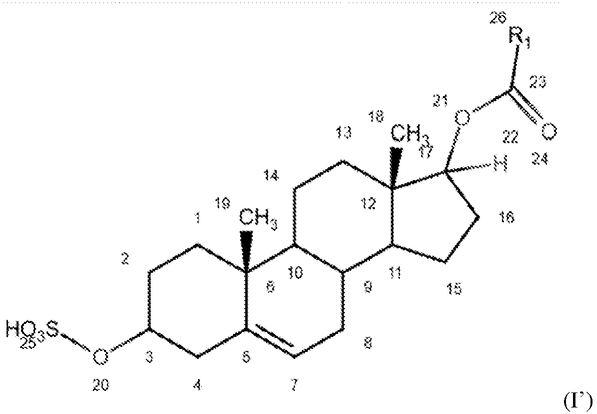 wherein R₁ is (CH₂)ₙ-CH₃,
wherein n is an integer from 6 to 12,
preferably n is 6, 8, 9, 10, 11 or 12,
more preferably n is 6, 8, 10 or 12, or
n is 8, 10 or 12,
highly preferably n is 10.

Thus, highly preferably R₁ is (CH₂)₁₀-CH₃.

In an embodiment the configuration at the 3^{rd} carbon is S (3β).

In a further embodiment the configuration at the 3^{rd} carbon is R (3α).

Preferably the configuration at the 3^{rd} carbon is S (3β).

In the present specification, alternatively, the IUPAC numbering of the sterane can be used, however, positions 3 and 17 are the same by IUPAC and in the present numbering shown.

Upon preparation of the compound a mixture of beta and alpha configuration in respect of carbon 3 may be present. In an embodiment, the mixture may be a racemic mixture. In an embodiment either the beta or the alpha configuration in respect of carbon 3 may be in excess.

In a pharmaceutical composition mixture of beta and alpha configuration in respect of carbon 3 may be present. In an embodiment, the mixture may be a racemic mixture. In an embodiment either the beta or the alpha configuration in respect of carbon 3 may be in excess.

It is to be noted that the 3β configuration is a particular configuration.

For example, the configuration of the compound can be as shown on general formula (Is1'):

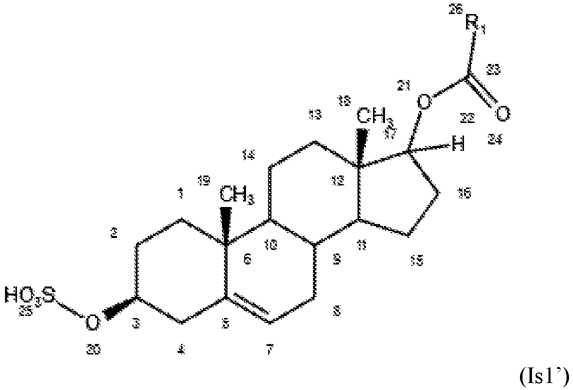

In an embodiment the configuration at the 17^{th} carbon is R.

In a further embodiment the configuration at the 17^{th} carbon is S.

In an embodiment the configuration at the 17^{th} carbon is beta (17β).

In a further embodiment the configuration at the 17^{th} carbon is alpha (17α).

In an embodiment the stereochemistry of the compound is selected from the group consisting of 17β,3β, 17β,3α, 17α,3β and 17α,3α, preferably 17β,3β.

Upon preparation of the compound a mixture of beta and alpha configuration in respect of carbon 17 may be present. In an embodiment, the mixture may be a racemic mixture. In an embodiment either the beta or the alpha configuration in respect of carbon 17 may be in excess.

In a preferred embodiment the configuration is 3β,17β as shown by Formula (Is2).

For example, the configuration of the compound can be as shown on general formula (Is2):

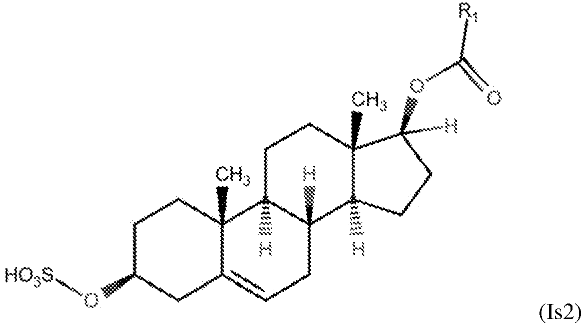

In a pharmaceutical composition mixture of beta and alpha configuration in respect of carbon 17 may be present. In an embodiment, the mixture may be a racemic mixture. In an embodiment either the beta or the alpha configuration in respect of carbon 17 may be in excess.

It is to be noted that the 17β configuration (which is present in DHEA) is a particular configuration.

The newly synthesized compound(s) were tested *ex vivo,* on isolated human neutrophil granulocytes and also in *in vivo* animal model. The present inventors chose Apo-E deficient mice kept on a lipid rich diet that are used as atherosclerosis models. The treatment lasted eight weeks. Control animals were given plain drinking water and lipid rich food, while the treated group, kept on the same diet, were administered 100 micrograms per animal per day of the new compound synthesized by the present inventors. The compound was dissolved in the drinking water. At the end of the examination period, the size and number of atherosclerotic plaques developed in the aortic arch and the inner surface of the aorta, calcification of heart valves, and serum hydrogen sulfide levels were determined. The present inventors found that in treated animals the number and size of plaques and the extent of calcification was significantly lower, and the concentration of hydrogen sulfide, a strong antioxidant was significantly higher. The latter may serve as explanation of the mechanism of action.

In the further part of the present work, it was examined whether the compounds of the invention have positive therapeutic effect, too. In the above-mentioned animal model, the present inventors developed during eight weeks real atherosclerosis, and for an additional eight weeks animals were given S2 compound (100 micrograms per day per animal). Control mice got pure drinking water. At the end of experiment the size and number of sclerotic plaques, the calcification of heart valves and the amount of hydrogen sulfide was measured. In animals treated with the compound of the invention the size and number of plaques and the calcification were significantly less, the concentration of hydrogen sulfide was significantly higher than in control animals. This means the compounds of the invention have not only preventive, but also therapeutic effect.

Without being bound by theory, the present inventors have studied the possible mechanism behind the biological and medical effect.

In previous studies it was found that elevated H₂S (I.) scavenged atherogenic free radical superoxide, (II.) limited the formation of pro-oxidant and pro-inflammatory lipid mediators and subsequent endothelial responses provoked by these species, (III.) prevented heart valves calcification by controlling osteoblast-like differentiation of valvular interstitial cells [Bekesi G, *et al.* 2000; Bekesi G, *et al.* 2004; Potor L, *et al.* 2018; Sikura KE, *et al.* 2019].

In a systematic study, it was found that neutrophils incubated with corticosterone and 18-hydroxy-deoxycorticosterone showed a significant reduction in superoxide production, whereas the present inventors found a significant enhancement in the presence of 11beta-hydroxyprogesterone. Furthermore, a non-significant decreasing trend was observed after incubation with cholesterol 3-sulphate and an increasing tendency using 11-hydroxyandrostenedione [Bekesi G, *et al.* 2004].

Hydrogen sulfide (H₂S) is the newest member of the endogenous gaseous transmitter family along with nitric oxide and carbon monoxide [Wang R. 2002]. Potor *et al.* also showed that H₂S exhibits an anti-atherosclerotic function in ApoE^{-/-} mouse on a high-fat diet via inhibiting lipid-peroxidation. Furthermore, they presented that, exogenous H₂S inhibited lipid oxidation of human atheroma and of human hemorrhage lesion [Potor *L, et al.* 2018]. Recently, Sikura and Potor *et al.* observed the inhibition of heart valves calcification by H₂S in ApoE^{-/-} mouse on a high-fat diet [Sikura KE, *et al.* 2019].

It has been found that an elevated level of H₂S in the mice plasma due to DHEA-derived steroid treatment contribute to this effect. In view of this the present inventors wanted to clarify whether the basic chemical structures (DHEA and its sulphate DHEAS) of the compound of the invention have antiatherogenic property, and found that in the animal model these basic compounds did not diminish the development of atherosclerosis but elevated mortality rate of the animals (see e.g. Figure 4C).

In conclusion, the present invention provides evidence that DHEA-derived steroid of the invention is potent preventive and therapeutic agent in arteriosclerosis as well as atherosclerosis and atherosclerosis-associated diseases.

The invention is useful in the treatment of a number of conditions and diseases.

### Atherosclerosis-associated diseases

Atherosclerosis is a pathological process in the arteries, in which the inside of an artery narrows due to the buildup of plaque. Arteries throughout the body can be affected, e.g., the coronary arteries, cerebral arteries, iliac and femoral arteries, and aorta, and it can cause various diseases, depending on which artery/arteries are affected [National Research Council (US) Committee on Diet and Health. (1989). Diet and Health: Implications for Reducing Chronic Disease Risk. National Academy Press, Washington D.C., 1989].

Lesions in the coronary arteries lead to CHD (coronary heart disease, also known as coronary artery disease = CAD), which is one of the most common and serious manifestations of atherosclerosis. The term coronary heart disease includes many syndromes, such as sudden cardiac death, myocardial infarction, stroke, heart attack, angina pectoris.

Sudden cardiac arrest or sudden cardiac death occurs when the electrical system to the heart malfunctions and becomes irregular. Blood flow to the body and to the brain is reduced, the affected person can lose consciousness and can die unless emergency treatment is begun immediately.

Myocardial infarction (or heart attack) occurs when blood flow decreases or stops to part of the heart, which causes ischemic necrosis of the myocardium. The necrotic tissue is replaced by connective tissue. The subsequent clinical outcome depends on the amount and location of the lost cardiac muscle.

The stenosis of the coronary arteries sometimes does not cause infarction, but it can cause ischemic pain, especially on exertion - this is called angina pectoris. This condition indicates the presence of severe lesions and high risk of myocardial infarction.

Stroke occurs when the flow of oxygen-rich blood to a portion of the brain is blocked. Without oxygen, the brain cells start to die, and the brain tissue becomes damaged. The two main types of stroke are ischemic (due to lack of blood), and hemorrhagic (due to bleeding). Thrombosis formed over an atherosclerotic plaque in a cerebral artery can decrease or interrupt blood flow to part of the brain, causing ischemic necrosis. The symptoms can be paralysis on the contralateral side, and disturbances of speech, vision, hearing, and memory.

Peripheral arterial disease (PAD) is an abnormal narrowing of arteries other than those that supply the heart or brain (e.g., abdominal aorta, iliac arteries, and femoral arteries). It occurs when atherosclerosis and its complications in the arteries produce temporary arterial insufficiency in the lower extremities or ischemic necrosis of the extremities. PAD includes several clinical syndromes of arterial insufficiency in the extremities, characterized by pain, inflammation, and ischemic damage to soft tissues from partial or complete occlusion of major arteries. PAD is often used interchangeably with lower extremity arterial disease (LEAD), when it affects the arteries of the lower extremities of the body.

Atherosclerosis can be associated with other diseases, as well.

Calcification can occur in atherosclerotic vascular lesions and in the aortic valve. Calcific aortic valve disease (CAVD) is a slow, progressive disorder. It has many forms from mild valve thickening, without obstruction of blood flow (aortic sclerosis) to severe calcification with impaired leaflet motion (aortic stenosis) [Lerman *et al.* 2015]. CAVD is the most common heart valve disease, and no medical treatment so far has been able to slow CAVD progression [Hulin *et al.* 2018].

Chronic kidney disease (CKD) can also be associated with atherosclerosis. The patients with CKD are at increased risk of atherosclerotic cardiovascular disease [Kon *et al.* 2014]. Accelerated atherosclerosis has been observed in early stages of renal dysfunction [Olechnowicz-Tietz *et al.* 2013]. A greater distinguishing feature of atherosclerotic cardiovascular disease in CKD is the severity of the disease, which is reflective of an increase in inflammatory mediators and vascular calcification secondary to hyperparathyroidism of renal origin that are unique to patients with CKD [Mathew *et al.* 2017].

Atherosclerosis can also be linked to vascular diseases in the eye. Retinal arteries may become blocked when a blood clot or fat deposits get stuck in the arteries. These blockages are more likely if there is atherosclerosis in the eye [Medical Encyclopedia, Retinal artery occlusion. Retrieved from https://medlineplus.gov/ency/article/001028.htm.]. If the central retinal artery is affected, i.e. the artery is narrowed, the blood supply to the inner retina can be reduced, which can cause loss of vision. Vascular hypertension can also occur in association with atherosclerosis, which can lead to vascular occlusions of the retina.

Atherosclerosis can also play a role in neurodegenerative diseases, such as Alzheimer's disease. According to Roher *et al.* [Roher *et al.* 2003] atherosclerosis-induced brain hypoperfusion contributes to the clinical and pathological manifestations of Alzheimer's disease. Alzheimer's disease is a progressive brain disorder, that causes a loss of brain cells that leads to memory loss and the decline of other thinking skills.

### Diseases associated with H₂S

H₂S was shown to exert various effects on the different systems, like the gastrointestinal, neuronal, cardiovascular, respiratory, renal, and hepatic systems [Singh, S. B., & Lin, H. C. 2015]. Hydrogen sulfide reduces arterial blood pressure, limits atheromatous plaque formation, and promotes vascularization of ischemic tissues. It has been shown that H₂S is a proangiogenic substance, which can restore ischemic tissue function, and it has a beneficial effect in treating cerebral artery occlusion, and post-ischemic cardiac remodelling, as shown in animal models [Kanagy, N. L., Szabo, C., & Papapetropoulos, A. 2017].

Hydrogen sulfide reduces the amount of reactive oxygen species (ROS) by inhibiting ROS production, by direct scavenging and by increased expression of antioxidant enzymes. Thus, H₂S can reduce oxidative stress and thereby counteract oxidative stress-related changes in the vessels, such as hypertension, atherosclerosis, and vascular diabetic complications [Kanagy, supra].

The present inventors have shown that treating ApoE^{-/-} mice with the DHEA-derived steroid S2 according to the invention increases H₂S levels in the serum, thus it can be reasonably expected to have beneficial effects in treating not just atherosclerosis-associated diseases, but other oxidative stress-related disorders or pathologies associated with decreased levels of H₂S.

According to the review of Zhang *et al.* (2013) [Zhang *et al.* 2013] H₂S is involved in aging as well, through by inhibiting free-radical reactions. Moreover, it has been shown that H₂S has a therapeutic potential in age-associated diseases [Kanagy, supra].

Antioxidant effects of hydrogen sulphide have been demonstrated in the context of atherosclerosis. H₂S delayed progression of the disease, and/or reduced its severity.

### Diagnosis of atherosclerosis and related disease

Atherosclerosis is preferably treated in patients diagnosed with atherosclerosis or preventive treatment is applied in patients at risk of arteriosclerosis or atherosclerosis.

Diagnosis of atherosclerosis includes measurement of a locally decreased blood pressure (near to the site of atherosclerotic lesions). Also changed blood flow may be heard by experts via stethoscope or a weak or absent pulse below the arterial area narrowed due atherosclerotic lesions can also be a sign of atherosclerosis.

Diagnosis of atherosclerosis may also include the following diagnostic tests:
blood tests;
ankle-brachial index, comparing blood pressure in the ankle with blood pressure in the arm;
electrocardiogram (ECG);
stress test (or exercise stress test) in which information is collected on the heart during physical activity;
moreover, imaging methods:
   ultrasonography or Doppler ultrasonography, non-invasive methods which enable differentiation of some plaque components,
   intravascular ultrasonography, which is invasive and radiation exposure and iodinated contrast agent is required for catheter positioning, the method is useful for plaque tissue characterization;
   optical coherence tomography, which is also invasive and radiation exposure and iodinated contrast agent is required for catheter positioning, the method provides a high-resolution imaging for plaque characteristics,
   computerized tomography (CT angiography) scan which enables differentiation between calcified and non-calcified plaque,
   MRI or Magnetic resonance angiography (MRA), a method limited essentially to large-calibre vessels and no evaluation of plaque components is possible [Libby, 2019].

Typically, therapeutic methods include diagnosis and once signs or symptoms of atherosclerosis is/are established therapy may begin.

Atherosclerosis begins decades before the appearance of its clinical consequences. As to the risk of arteriosclerosis and atherosclerosis, it is advisable to start preventive treatment in patients having a significant risk of developing these diseases.

Risk factors typically include obesity, diabetes mellitus type II, elevated low-density lipoprotein (LDL) cholesterol levels, metabolic syndrome and family history of atherosclerosis or associated disease all present, by way of example, increased risk factors. As to life management a sedimentary lifestyle, smoking, cholesterol-rich diet etc. present further risk factors.

While guidelines may differ per countries on diagnosis and may change in time, virtually all guidelines recommend the initial evaluation of individual risk of future cardiovascular disease (CVD) [Libby, 2019]. It is within the skills of a medical doctor to decide on preventive treatment which may be started once risk of development of arteriosclerosis and/or atherosclerosis exists.

Dose of preventive treatment may be lower than those of therapeutic treatment and length of administration may be longer.

In case when atherosclerosis has been manifested and cannot be reversed fully, the treatment may have a therapeutic feature in that an effort is made to reverse, at least partially, the lesions or plaques formed, and a preventive feature to avoid further lesions or plaques to be formed.

In certain cases, in particular if the risk factors support this decision and/or if no full reversal of the symptoms is achieved, the medical doctor responsible for the treatment may decide that long term treatment is necessary. Aging provides an additional risk factor, e.g. in case of patients above 40 years or above 50 years or in particular above 55 years or above 60 years long term treatment may be justified.

Long term treatment may mean administration of the compound or pharmaceutical composition of the invention for at least 6 months, or for at least 1 year, 2 years, 5 years or lifelong.

### Pharmaceutical compositions

In the present invention the stereoisomers, enantiomers or diastereomers may be present in the pharmaceutical compositions. In preferred compositions the active isomers are enriched. Various isomers are disclosed hereinabove and in the examples.

Pharmaceutically acceptable salts may be obtained by treating a compound with a corresponding base or salt of the cation to be added to the composition.

The compounds of the invention may exist in unsolvated as well as in solvated forms with pharmaceutically acceptable solvents such as water. In general, the solvated forms are considered equivalent to the unsolvated forms for the purpose of the invention.

As to pharmaceutically acceptable carriers and excipients, the term acceptable means being compatible with the other ingredients of the composition and not deleterious to the recipients thereof.

For oral administration, the active ingredient may be presented in a form as described in the Brief description of the invention section.

In general, preparation of a composition with a pharmaceutically acceptable carrier or excipient may be carried out as described in e.g. as a standard reference, Gennaro, A.R. et al., Remington: The Science and Practice of Pharmacy (20th Edition., Lippincott Williams & Wilkins, 2000, see especially Part 5: Pharmaceutical Manufacturing).

More specifically, oral lipid-based drug delivery systems can e.g. be prepared as described in the review paper Kalepu, S, Oral lipid-based drug delivery systems - an overview. Acta Pharmaceutica Sinica B, 2013 3(6) 361-372.

Solubilization of oral and injectable formulation is taught e.g. in Strickley, R.G. Solubilizing Excipients in Oral and Injectable Formulations. Pharmaceutical Research, Vol. 21, No. 2, February 2004.

The pharmaceutical composition of the invention may be presented in unit-dose or multi-dose containers.

In case of oral preparation, solid dosage units, such as pills, tablets, etc. as given above, can be provided.

Injection liquids can be presented e.g. in predetermined amounts, for example in sealed vials and ampoules, and may also be stored in the form of dry crystals or freeze dried (lyophilized) condition requiring only the addition of sterile liquid carrier, e.g. water or physiological salt solution, prior to use.

The compounds of the present invention are present in the pharmaceutical compositions in a purity appropriate for human or animal administration. Purification techniques include e.g. recrystallization as shown in the examples and/or chromatographic purification as well known in the art.

The present invention is further illustrated by way of non-limiting examples.

### EXAMPLES

### MATERIALS AND METHODS

All reagents were purchased from Sigma-Aldrich (Darmstadt, Germany) with ACS grade or higher purity, exceptions are specified below including DHEA. Steroid compounds were taken up in tap-water followed by sonication. DHEA-derived steroid was produced by the inventors as described in Examples. The reagents used to synthesis were from Reanal, Budapest, Hungary ("alt" quality).

### Mice

All the animal experiments were approved by the guidelines from Directive 2010/63/EU of the European Parliament on the protection of animals used for scientific purposes. Animal experiments performed in this study were approved by the Scientific and Research Ethics Committee of the Scientific Council of Health of the Hungarian Government under the registration number of DE MÁB/157-5/2010 and are reported in accordance with the ARRIVE guidelines. C57BL/6 ApoE^{-/-} mice (The Jackson Laboratory; B6.129P2 Apoetm1Unc/J; RRID:IMSR_JAX:002052) were maintained at the University of Debrecen under specific pathogen-free conditions in accordance with guidelines from Institutional Ethical Committee. To induce atherosclerotic plaque formation, standard chow diet was changed to atherogenic diet (15% fat, 1.25% cholesterol, ssniff Spezialdiäten GmbH, Soest, Germany) at the age of 8 weeks. Mice were randomly divided into two groups and parallelly with the atherogenic diet, mice received tap-water or DHEA-derived steroid (100 µg steroid/mouse/day) in tap-water. The DHEA-derived steroid was replaced every day. Aortas were harvested after 8 weeks of treatment. All mice were euthanized by a predictable and controllable administering of slow-fill compressed CO₂ asphyxiation. Atherogenic food composition (high-fat diet): Crude Nutrients (%): Crude Protein 19%; crude fat 15.2%; crude fiber 3.4%; crude ash 6.3%; starch 25.6%; sugar 11.2%; Additives (per kg): vitamin A 15,000 IU; vitamin D3 1,000 IU; vitamin E 110 mg; vitamin K3 5 mg; vitamin C 0 mg; copper 13 mg.

### Quantification of aortic lesions in ApoE^{-/}⁻ mice

Aortas were dissected from the aortic arch to the iliac bifurcation. After that, the fatty tissue was carefully removed under a stereomicroscope (M125; Leica Biosystems Nussloch GmbH). The atherosclerotic lesions were revealed by *en face* Oil Red O (Sigma-Aldrich, Lot. O0625;) staining. 0.07 g ORO powder was dissolved in 25 mL methanol and mixed for 10 min on a magnetic stirrer. Finally, 10 mL 1 mol/L NaOH was added to the ORO solution and filtered it. Images were taken with the camera (Nikon D3200; Nikon Corp., Japan) and whole aorta plaque area was analyzed with ImageJ 1.49 software.

### Immunohistochemistry

Immunohistochemistry from the aortic root was performed on formalin-fixed, paraffin-embedded tissue sections. 4 µm slides were then deparaffinated using xylol and ethanol. The serial sections were stained with Gill Hematoxylin solution (Merck) followed by eosin counterstaining or with von Kossa. For digital documentation, the stained specimens were scanned with a Mirax Midi scanner (3D Histech, Budapest, Hungary). Macroscopic pictures of the arteries were taken with Nikon D3200 camera (Nikon Corp.; Minato, Tokyo, Japan).

### Determination of sulfide level from tissue with zinc precipitation assay

Sulfide levels were measured with zinc precipitation method based upon developed by Gilboa-Garber [Gilboa-Garber N. 1971] and modified by A. D. Ang *et al.* [Ang AD, *et al.* 2012]. The human carotid artery was homogenized under liquid nitrogen in 7.4 pH PBS and sonicated it. After sonication the sample was centrifuged at 12000 G for 15 min and the lipids free clear supernatant was collected. 200 µL sample was mixed with 350 µL 1% zinc acetate and 50 µL 1.5 mol/L sodium hydroxide and incubated for 60 minutes on a shaker. Incubation step was followed by centrifugation at 2000 G for 5 minutes to pellet the generated zinc sulfide. The supernatant was then removed, and the pellet washed with 1 mL of distilled water by vortexing extensively, followed by centrifugation at 2000 G for 5 minutes. The supernatant was then aspirated off and the pellet reconstituted with 160 µL of distilled water and mixed with 40 µL of pre-mixed dye (20 µL of 20 mmol/L dimethyl-p-phenylenediamine dihydrochloride (NNDP) in 7.2 mol/L hydrochloric acid (HCl) and 20 µl of 30 mmol/L Iron(III) chloride (FeCl₃) in 1.2 mol/L HCl). After 10 min the absorbance of the generated methylene blue (MB) was measured with a spectrophotometer at 667 nm. Since during the reaction 1 mol/L MB formed from 1 mol/L sulfide, the concentration was determined by the MB's extinction coefficient (30 200 M⁻¹cm⁻¹). Samples were normalized for protein concentration.

### Experimental units

"N" represents the number of tissue samples used in each group. The "n" denotes the number of replications of the independent results.

### Statistical analysis

Data were analyzed by GraphPad Prism 5.02 software (GraphPad Software Inc., 7825 Fay Avenue, Suite 230 La Jolla, CA 92037; RRID: SCR_002798). All statistical data are expressed as mean ± SEM. If data groups passed the normality test and equal variance test, we performed Student's t-test or One Way ANOVA followed by Bonferroni post hoc tests as indicated in the Brief description of the figures. P<0.05 was considered significant.

### Quantification of DHEAS in biological sample

DHEAS can be quantitatively determined in biological samples as described e.g. by Sánchez-Guijo *et al.* [Sánchez-Guijo *et al.,* 2015] and papers referenced by them.

### PREPARATION OF THE COMPOUNDS OF THE INVENTION

Name of the compounds prepared:
**A) (n = 4):**
   17-hexanoyloxy-5-androstene-3-sulphate sodium salt
**B) (n = 10):**
   17-lauroyloxy-5-androstene-3-sulphate sodium salt
**C) (n = 14):**
   17-palmitoyloxy-5-androstene-3-sulphate sodium salt

### Molecular formulas and molecular weights:

| | | |
|---|---|---|
| **A):** n=4; | C₂₅H₃₉NaO₆S; | MW: 490.6 |
| **B):** n = 10; | C₃₁H₅₁NaO₆S; | MW: 574.8 |
| **C):**n = 14; | C₃₅H₅₉NaO₆S; | MW: 630.9 |

| | |
|---|---|
| **Structure:** | 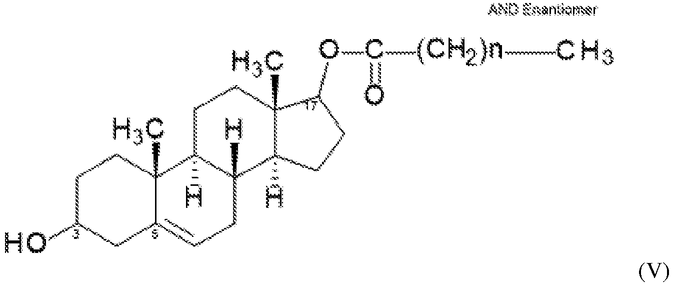 |
| **Solubility:** | Ethanol-water, methanol water mixtures |
| **Notes:** | During synthesis reactions were monitored by thin layer chromatography (TLC). The compounds prepared were identified by H¹NMR and IR spectroscopy |

### Reaction pathway

### Preparation of 17-lauroyloxy-5-androstene-3-sulphate sodium salt (compound S2)

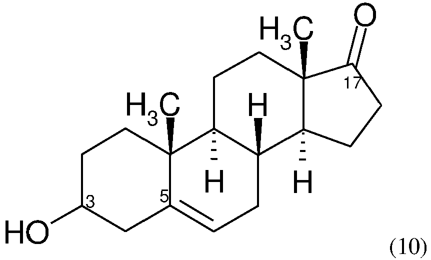

C₁₉H₂₈O₂
MW = 288.4

### 3-hydroxy-17-oxo-5-androstene (DHEA) (10)

| | |
|---|---|
| **I. Acylation** | 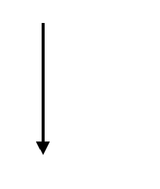 |
| R: (CH₃CO)₂O | |
| Solvent: dichloromethane | |
| Temperature: 0°C | |
| Reaction time: 3 hours | |

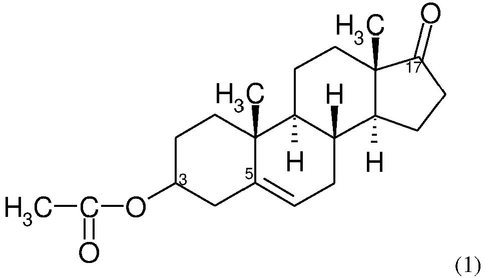

C₂₁H₃₀O₃
MW = 330.46

### 3-acetoxy-17-oxo-5-androstene (1)

| | |
|---|---|
| **II. Reduction** | 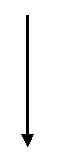 |
| R: NaBH₄ | |
| Solvent: methanol | |
| Temperature: (-)5°C | |
| Reaction time: 2.5 hours | |

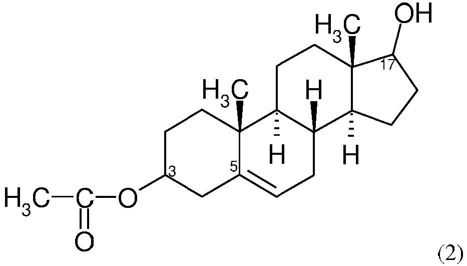

C₂₁H₃₂O₃
MW = 332.5

### 3-acetoxy-17-hydroxy-5-androstene (2)

| | |
|---|---|
| **III. Acylation** | 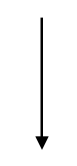 |
| R: CH₃(CH₂)₁₀-COCl | |
| Solvent: dichloromethane, pyridine | |
| Temperature: 25°C | |
| Reaction time: 8 hours | |

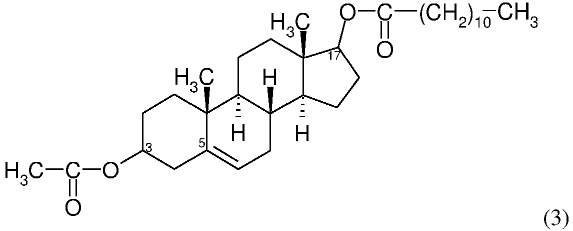

C₃₃H₅₄O₄
MW = 514.8

### 3-acetoxy-17-lauroyloxy-5-androstene (3)

| | |
|---|---|
| IV. **Hydrolysis** | 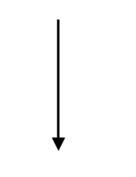 |
| R: NaOH | |
| Solvent: methanol/water | |
| Temperature: 20°C | |
| Reaction time: 12 hours | |

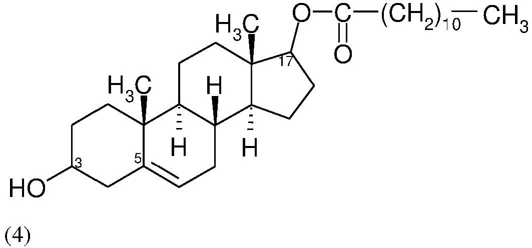

C₃₁H₅₂O₃
MW = 472.7

### 3-hydroxy-17-lauroyloxy-5-androstene (4)

| | | |
|---|---|---|
| V. **Sulphation** | 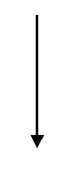 | VI. **Salt formation** |
| R: ClSO₃H | | R: NaHCO₃ |
| Solvent: dichloromethane/pyridine | | Solvent: water |
| Temperature: (-)5°C | | Temperature: 20°C |
| Reaction time: 4 hours | | Reaction time: 0.5 hour |

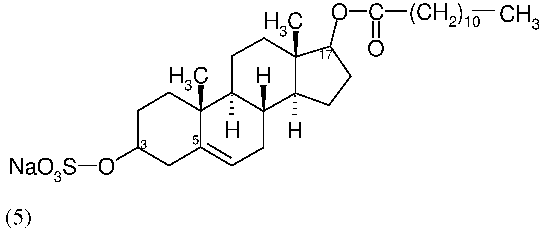

C₃₁H₅₁O₆NaS
MW = 574.8

### 17-lauroyloxy-5-androstene-3-sulphate sodium salt (5)

### Synthesis of 17-lauroyloxy-5-androstene-3-sulphate sodium salt

### I. 3-acetoxy-17-oxo-5-androstene (1)

Into a round bottom flask with magnetic stirrer 25 cm³ dichloromethane (DCM) and 5.8 g (20 mmol) DHEA was measured and added. The solution was cooled to T = 0°C and a mixture of 10 cm³ dichloromethane, 3.1 g acetic anhydride and 200 µl conc. H₂SO₄ was added drop by drop. The mixture was stirred for 3 hours (t = 3 hours) then poured into 50 cm³ 5 % (w/v) NaHCO₃ solution (T = 5°C) and stirred for 10 min; thereafter further 50 cm³ DCM was added. The organic phase was washed with water then with 20% (w/v) NaCl solution and dried over Na₂CO₃. The solvent was removed in vacuum (Rotadest). The product was stirred with a mixture of 50 cm³ methanol and 50 cm³ water for 15 min, then cooled to T = 0°C.

The white crystals of *3-acetoxy-17-oxo-5-androstene* (compound I) were filtered out and washed with a small amount of cold solvent then with water and then dried in a vacuum desiccator at T = 50°C.
Yield: = 5.2 g; R = 78.8%

### II. 3-acetoxy-17-hydroxy-5-androstene (2)

Compound I (4.96 g) and 180 cm³ methanol was added to a flask with magnetic stirrer and cooled to T = -5°C. To this cooled solution 0.7 g sodium-borohydride was added in multiple parts during about 30 min. Thereafter the mixture was allowed to react for a further 2.5 hours and then the reaction mixture was mixed with 100 cm³ 0.4 M cold hydrochloric acid (HCl). The mixture so obtained was neutralized to pH 6 by 1 M NaHCO₃ (about 25 cm³) and diluted by further 50 cm³ water. The product was filtered out, then washed with 2x15 cm³ water:methanol (1:1) mixture and with 2x25 cm³ water.
After vacuum drying (T = 50°C) 4.1 g product was obtained (R = 82.0%).

### III. 3-acetoxy-17-lauroyloxy-5-androstene (3)

Into a flask with magnetic stirrer, 20 cm³ DCM, 15 cm³ pyridine, 3.35 g (10 mmol) 3-acetoxy-17-hydroxy-5-androstene (compound II) were measured and added, then at T = 20-25°C, during 20-30 min, a mixture of 3.1 g (14 mmol) lauric acid chloride (lauroyl chloride) and 10 cm³ DCM was added.

Thereafter the mixture was allowed to react for an additional 5.0 hours (t = 5.0 hours), then the mixture was diluted by 50 cm³ DCM and mixed with 50 cm³ 10% hydrochloric acid at T = 5-10°C. The separated organic phase was washed twice with 10% hydrochloric acid, dried by sodium sulphate, and then the solvent was removed in a rotary evaporator (Rotadest) at reduced pressure in water-bath at T = 55°C.

The crude product was suspended by 40 cm³ water:MeOH (1:1) mixture at T = 55-60°C, then cooled to T = 5°C, and then the crystals were filtered out and washed with 2 x 10 cm³ of the aforementioned solvent.
After vacuum drying (T = 50°C), 4.3 g of compound III was obtained (R = 83.4%).

### IV. 3-hydroxy-17-lauroyloxy-5-androstene (4)

3.1 g (6.0 mmol) of compound III obtained in the previous step was dissolved in 120 cm³ methanol at room temperature, then 5.4 cm³ 1 M NaOH (5.4 mmol) was added, and the mixture was stirred for 12 hours. Then 60 cm³ water was added to the mixture and the reaction mixture was acidified to pH 6 by 2 M hydrochloric acid. The precipitated product was filtered out and washed with 20 cm³ water:MeOH (1:1) mixture and then with 2 x 20 cm³ water. The wet material was dissolved in 50 cm³ chloroform, extracted (by shaking) with 2 x 20 cm³ water, and then the solution was dried by Na₂SO₄, and then the solvent was removed in Rotadest. The product was dried in vacuum dryer (T = 50°C). 2.30 g of compound IV was obtained (R = 80.0 %).

### V. 17-lauroyloxy-5-androstene-3-sulphate sodium salt (5)

Into a flask with magnetic stirrer, 25 cm³ DCM, 10 cm³ pyridine and 1.42 g (3 mmol) 3-hydroxy-17-lauroyloxy-5-androstene (compound IV) were measured and added. The reaction mixture was cooled to T = -5°C, and then 0.420 g (250 µl) chlorosulfonic acid (sulfurochloridic acid) mixed with 5 cm³ cool dichloromethane was added to the mixture. After allowing to react for 4.0 hours, 20 cm³ 5% sodium bicarbonate solution and 20 cm³ water was added to the mixture at room temperature, then the mixture was stirred vigorously for 30 min.

The three-phase reaction mixture was diluted by 50 cm³ diisopropyl ether and after 15 min stirring the mixture was filtered. The filtered out crude product was dried in vacuum dryer (T = 50°C), then stirred for 30 min at T = 50-55°C. After cooling it down, the target product was filtered out, washed with 2 x 10 cm³ water, and then again dried in vacuum.

The dry product was suspended by 50 cm³ DCM at T = 40°C, and after cooling was filtered out and on the filter it was washed again twice with 15 cm³ DCM. Again, it was dried in a vacuum dryer. 1.1 g product was obtained, R = 63.9%.

NMR spectrum of the compound is shown in Figure 7B.

**The other two related compounds (compounds C6 and C16)** were also obtained in the outlined reaction pathway. In these cases, at first the acylation of intermediate II with hexanoyl chloride or palmitoyl chloride, respectively, was carried out.

### Synthesis of 17-hexanoyloxy-5-androstene-3-sulphate sodium salt (comparative compound C6)

### Acylation

### Preparation of 3-acetoxy-17-hexanoyloxy-5-androstene

| | | |
|---|---|---|
| Molecular formula: | C₂₇H₄₂O₄ | MW: 430.6 |

Into a flask with magnetic stirrer, 25 cm³ DCM (or chloroform), 20 cm³ pyridine and 4.0 g (12 mmol) 3-acetoxy-17-hydroxy (II) intermediate were measured and added. After that, during cooling it by cool water, 2.16 g (16 mmol) hexanoyl chloride mixed with 10 cm³ solvent was added drop by drop at T = 20°C, during about 30 min.

After allowing the mixture to react for 3 hours, the reaction mixture was diluted by 50 cm³ solvent and was added to V = 150 cm³ 10% hydrochloric acid aqueous solution at T = 5-10°C during about 30 min (vigorous stirring!), and then after stirring for 15 min the phases were separated. The organic phase was washed twice with water and once with 10% saline, then dried by sodium sulphate, and then the solvent was removed in Rotadest at reduced pressure.

The product was suspended by 50 cm³ water:MeOH (1:1) mixture at T ≈ 55°C, then cooled down to T ≈ 5°C and was filtered. The filter cake of crystals was further washed with 2 x 15 cm³ solvent.

After vacuum drying (T = 50°C), 4.1 g product was obtained (R = 79.3%).

### 1) Hydrolysis

### Synthesis of 3-hydroxy-17-hexanoyloxy-5-androstene

| | | |
|---|---|---|
| Molecular formula: | C₂₅H₄₀O₃ | MW: 388.5 |

Into a flask with magnetic stirrer, 200 cm³ methanol and 3.9 g (9 mmol) of diacyl derivative prepared in the previous step were measured and added at T = 22°C. Then during about 30 min with vigorous stirring 8.2 cm³ c = 1M sodium hydroxide solution was added drop by drop. After stirring for 4 hours, 100 cm³ water and 20 cm³ c = 0.4M HCl solution were added to the reaction mixture. After stirring for 15 min, pH was adjusted to pH = 6.5-7.0 by 5% NaHCO₃.

The product was filtered out and was washed with 2 x 25 cm³ water:MeOH (1:1) mixture and then with 25 cm³ water.

The wet material was dissolved in 50 cm³ chloroform and extracted by shaking with 2 x 25 cm³ water, then dried by sodium sulphate, and then the solvent was removed in Rotadest at reduced pressure and in water-bath at T ≈ 55°C. Finally, it was dried in vacuum dryer (T = 50°C).

2.6 g product was obtained (R = 74.2%).

### 2) Sulphation and salt formation (compound C6)

Into a flask with magnetic stirrer, to a solution of 2.34 g (6 mmol) 3-hydroxy-17-hexanoyloxy-5-androstene dissolved in chloroform (25 cm³), 2.5 cm³ pyridine was added. The reaction mixture was cooled down to T = 0 - (-5)°C and during about 20-30 min a solution of 880 mg chlorosulfuric acid and T < 0°C chloroform was added drop by drop.

Thereafter, the mixture was allowed to react for an additional 4.0 hours at T ≈ 5°C, and then 35 cm³ 5% sodium bicarbonate solution and 20 cm³ water, and 50 cm³ diisopropyl ether were slowly added. The heterogeneous reaction mixture was stirred for 15 min, and the solid product was filtered out and then dried in vacuum dryer (T = 50°C). Then the material was stirred with 20 cm³ water for 30 min at T = 50-55°C.

After cooling it down, the target product was filtered out and washed with 2 x 10 cm³ water, and then again dried in vacuum dryer.

The dry crystallic material was stirred with 50 cm³ DCM at T = 40°C for 30 min, then after cooling it down was filtered out and washed with 2 x 15 cm³ solvent, and then again dried.

1.8 g of 17-hexanoyloxy-5-androstene-3-sulphate sodium salt was obtained (R = 61.2%).

NMR spectrum of the compound is shown in Figure 7A.

### Synthesis of 17-palmitoyloxy-5-androstene-3-sulphate sodium salt (comparative compound C16)

### 1) Acylation

### Synthesis of 3-acetoxy-17-palmitoyloxy-5-androstene

| | | |
|---|---|---|
| Molecular formula: | C₃₇H₆₂O₄ | MW: 570.8 |

In a flask with magnetic stirrer, in a mixture of 15 cm³ chloroform and 10 cm³ pyridine 3.0 g (9 mmol) 3-acetoxy-17-hydroxy-5-androstene was dissolved, and during cooling down, in about 30 min, at T = 20°C, a mixture of 10 cm³ chloroform and 3.3 g (12 mmol) palmitoyl chloride was added.

After stirring for an additional 5.0 hours, the reaction mixture was diluted by 25 cm³ chloroform, and then it was added to V = 100 cm³ 10% hydrochloric acid solution at T = 5-10°C, during vigorous stirring. Then the separated organic phase was washed twice with water and then was extracted by shaking once with 10% sodium chloride solution. The mixture was dried by sodium sulphate and the solvent was removed in Rotadest at reduced pressure (water-bath, T ≈ 50°C).

The crystallic product was suspended by 50 cm³ water:MeOH (1:1) mixture, then cooled down to T < 10°C, and then it was filtered out and washed with 2 x 10 cm³ solvent.

After vacuum drying (T = 50°C), 3.9 g product was obtained (R = 75.9%).

### 2) Hydrolysis

### Preparation of 3-hydroxy-17-palmitoyloxy-5-androstene

| | | |
|---|---|---|
| Molecular formula: | C₃₅H₆₀O₃ | MW: 528.7 |

Into 300 cm³ methanol, 3.42 g (6.0 mmol) diacyl compound was measured and added, and then at first the suspension was heated to T = 55°C, and then swiftly cooled down to T ≈ 20-22°C. After that, during about 30 min, a mixture of 5.4 cm³ c = 1.0 M sodium hydroxide and 10 cm³ methanol was added drop by drop.

The reaction mixture was stirred for 36.0 hours at room temperature. During this long period, 85-90% conversion can be reached. Allowing to react for an additional time is inadvisable as it would produce unwanted by-product, and the remaining ~15% starting material does not hamper the final sulphation reaction.

After the hydrolysis, 100 cm³ water and 20 cm³ c = 0.4 M hydrochloric acid were added to the reaction mixture. Then, about 80% of the methanol was distilled in Rotadest at reduced pressure in water-bath at T ≈ 55°C. The suspension was cooled down, filtered, and the solid product was washed with 2 x 20 cm³ water:MeOH (1:1) mixture and then with 2 x 20 cm³ water.

After vacuum drying, 3.0 g, ~ 85% product was obtained (~2.55 g active substance; R: ≈ 80%).

### 3) Sulphation and salt formation (compound C16)

Into the usual equipment, 15 cm³ chloroform, 2.0 cm³ pyridine and 2.4 g (~4 mmol) 17-hydroxy derivative were measured and added. The mixture was cooled down to T = 0 - (-5)°C, and then during 20-30 min a mixture of 10 cm³ T ≈ 0°C chloroform and 590 mg chlorosulfuric acid was added drop by drop.

After that, the mixture was stirred for 5 hours at T ≈ 5°C, and then 25 cm³ 5% sodium bicarbonate solution and 20 cm³ water, and 40 cm³ diisopropyl ether were added slowly. The three-phase mixture was stirred for 15 min, and then the product was filtered out and then dried in vacuum dryer (T = 50°C).

After that, the crude product was stirred with 20 cm³ water at T = 50-55°C for 30 min, and after cooling it down, the product was filtered out and washed with 2 x 10 cm³ water. The product was dried in vacuum dryer (T = 50°C). The dry crystallic material was stirred with 50 cm³ DCM at T = 40°C for 30 min, then cooled down and then was filtered out and washed with 2 x 15 cm³ solvent.

After vacuum drying, 1.5 g of 17-palmitoyloxy-5-androstene-3-sulphate sodium salt was obtained (R = 59%).

NMR spectrum of the compound is shown in Figure 7C.

### RESULTS

### DHEA-derived steroid abrogates atherosclerotic plaque formation in mice

In order to investigate, whether DHEA-derived steroid has an anti-atherogenic effect, the present inventors performed experiments with an atherosclerotic animal model. DHEA-derived steroid drinking mice were sacrificed after 8 weeks and aortas were dissected from *aortic arch* to *iliac bifurcation.* The formation of atherosclerotic lesion was revealed by lipid staining. As *en face* Oil Red O staining of the whole aorta presented by Figure 1A, administration of DHEA-derived steroid solution inhibited atherogenic diet-induced atherosclerotic plaque formation in ApoE^{-/-} mice compared to tap water drinking (control) mice (Figure 1A). Quantification of Oil Red O staining of the whole aorta in ApoE^{-/-} mice showed a significantly lesser area of atherosclerotic lesion in DHEA-derived steroid group (N=9) as compared to the control group (N=13) (Figure 1B).

### DHEA-derived steroid prevents heart valve calcification

ApoE^{-/-} mice on a high-fat diet exhibited an expansion of extracellular matrix deposition in aortic valves. As demonstrated by von Kossa staining, calcific nodules appearing in the aortic heart valves of ApoE^{-/-} mice have been provoked by high-fat diet (Figure 2A; upper-middle and upper-right panels). In order to demonstrate the anti-calcification benefit of DHEA-derived steroid *in vivo,* DHEA-derived steroid was added to the drinking water of mice. Following this the valvular calcification was assessed. The present inventors concluded that DHEA-derived steroid treatment significantly inhibited the development of calcific nodules in aortic valves (Figure 2A; lower-middle and lower-right panels). Analysis of the aortic valves calcification nodules area showed a 91.2% lower mineralized area in mice treated with DHEA-derived steroid compared to ApoE^{-/-} mice drinking tap-water (Figure 2B).

### DHEA-derived steroid treatment increases H₂S levels in mouse serum

The present inventors' research group recently revealed, that H₂S has a strong ability to inhibit atherosclerotic plaque formation and calcification via attenuation inflammation, as well as prevention LDL oxidation, scavenging reactive oxygen species [Potor L, *et al.* 2018; Sikura KE, *et al.* 2019]; and reversing aging-associated pathologies [Perridon BW, *et al.* 2016; Zhan J-Q, *et al.* 2018; Hou CL, *et al.* 2016]. Thus, the present inventors measured the H₂S presence in the mice plasma derived from tap-water drinking or DHEA-derived steroid treated ApoE^{-/-} mice. They found that DHEA-derived steroid treated mice plasma's H₂S concentration was approximately 2.4 times higher compared to the control mice plasma (Fig. 3).

### DHEA, DHEA-S, C6 and C16 are not able to abrogate atherosclerotic plaque formation in mice

To investigate the specific inhibitor effects of the S2 steroid in atherosclerosis, the present inventors performed animal experiments with the basic molecule of S2 steroid derived from DHEA. Additionally, to further investigate the characteristic of S2 the present inventors examined the sulfide side chain of S2 using Dehydroepiandrosterone sulfate (DHEA-S). Finally, the present inventors also investigated the length of carbon side chains of S2 in atherosclerosis using 17-hexanoyloxy-5-androsten-3-sulfate sodium salt (C6) and 17-palmitoyloxy-5-androsten-3-sulfate sodium salt (C16) steroid. The present inventors found that, neither DHEA (N=11), DHEA-S (N=7), C6 (N=3) and nor C16 (N=3) molecules can abrogate atherosclerotic plaque formation in mice (Figure 4A and B). Furthermore, the present inventors found significant mouse death in experiments with DHEA (45.5%) and DHEA-S (28.6%) (Figure 4C).

### DHEA and DHEA-S failed to prevent calcification of aortic valve of ApoE knock out mice

The present inventors investigated the DHEA and DHEA-S effects on calcification of mouse heart valves. The present inventors found that neither DHEA nor DHEA-S is able to inhibit mineralization of aortic valve compared to S2 steroid (Figure 5A and B).

### The extent of atherosclerotic lesion and valvular aortic calcification already developed in ApoE knock out mice is decreased by employing DHEA-derived steroid S2

From a pharmacological point of view to the best of inventors' knowledge, there is no medicine in the market to reverse atherosclerosis and calcification of vessels. Current drugs only slow down the progression of atherosclerosis. Therefore, the present inventors examined the possibility of S2 steroid treatment to reverse the already formed atherosclerotic lesion and calcification. In this experiment the present inventors kept the mice on atherogenic diet for 4 or 6 weeks for development of atherosclerotic lesion and calcification. After that the present inventors changed the atherogenic diet to chop diet and they added S2 steroid to the mice's water for 4 weeks and 8 weeks. Control group got tap water only. Surprisingly the present inventors observed significantly lesser atherosclerotic lesion in case of S2 therapy (Figure 6A-D). Also, significant lesser extracellular matrix mineralization was observed using S2 therapy (Figure 6E and F).

### INDUSTRIAL APPLICABILITY

Despite the improving tendency in the past few years, diseases developing on the basis of atherosclerosis (myocardial infarct, stroke, obliterating lower extremity artery disease, Alzheimer's disease, circulation problems of the eye) still lead the morbidity and mortality statistics worldwide. Considering that billions of people are affected by these diseases, after a possibly successful medical innovation of the new compound of the present inventors, it could be a drug used both preventively and therapeutically without endocrinological side effect. The human, scientific, professional, and not least of all economic impact is hardly evaluable.

The present study demonstrates that DHEA-derived S2 steroid represents a novel atheroprotective drug, usable for preventing the formation of lipid derivatives in the atherosclerotic plaque and inhibits calcification of heart valves. Based on the present study, DHEA-derived steroid (S2) induced elevated H₂S (I.) scavenged atherogenic free radical superoxide, (II.) limited the formation of pro-oxidant and pro-inflammatory lipid mediators and subsequent endothelial responses provoked by these species, (III.) prevented heart valves calcification by controlling osteoblast-like differentiation of valvular interstitial cells.

In conclusion, the present study provides evidence that DHEA-derived steroid is a potent preventive and therapeutic agent in atherosclerosis and aortic valve calcification.

### REFERENCES

Ang AD, Konigstorfer A, Giles GI, et al. Measuring free tissue sulfide. Advances in Biological Chemistry 2012; 02: 360-365.
Aragno M. et al. Dehydroepiandrosterone pretreatment protects rats against the pro-oxidant and necrogenic effects of carbon tetrachloride. Biochem Pharmacol 1993; 46, 1689 -94
Bekesi G, Kakucs R, Varbiro S, et al. In vitro effects of different steroid hormones on superoxide anion production of human neutrophil granulocytes. Steroids 2000; 65: 889-894.
Bekesi G, Racz K, Hrabak A, et al. Systematic investigation of different steroid precursors with respect to their effect on superoxide anion production by human neutrophil granulocytes. Horm Metab Res 2004; 36: 155-163.
Brancaleone V. et al. Crucial role of androgen receptor in vascular H2S biosynthesis induced by testosterone. British Journal of Pharmacology (2015) 172 1505-1515 1505
Chester A. Molecular and cellular mechanisms of valve calcification. 2011; 2011: 4.
Gennaro, A.R. et al., Remington: The Science and Practice of Pharmacy (20th Edition., Lippincott Williams & Wilkins, 2000, see especially Part 5: Pharmaceutical Manufacturing)
Gilboa-Garber N. Direct spectrophotometric determination of inorganic sulfide in biological materials and in other complex mixtures. Analytical Biochemistry 1971; 43: 129-133.
Hochberg et al. Steroidal Fatty Acid Esters J.Steroid Bichem. molec. Biol. (1991) 40(4-6) pp577-585
Hou CL, Wang MJ, Sun C, et al. Protective Effects of Hydrogen Sulfide in the Ageing Kidney. Oxid Med Cell Longev 2016; 2016: 7570489.
Hulin, A et al. (2018). Advances in Pathophysiology of Calcific Aortic Valve Disease Propose Novel Molecular Therapeutic Targets. Frontiers in cardiovascular medicine, 5, 21.
Insull, W. The Pathology of Atherosclerosis: Plaque Development and Plaque Responses to Medical Treatment. The American Journal of Medicine 122(1), Suppl., S3-S14, 2009
Kalepu, S, Oral lipid-based drug delivery systems - an overview. Acta Pharmaceutica Sinica B, 2013 3(6) 361-372.
Kanagy, N. L., Szabo, C., & Papapetropoulos, A. (2017). Vascular biology of hydrogen sulfide. American journal of physiology. Cell physiology, 312(5), C537-C549.
Kon, V., Linton, M. F., & Fazio, S. (2011). Atherosclerosis in chronic kidney disease: the role of macrophages. Nature reviews. Nephrology, 7(1), 45-54.
Lerman et al. (2015). Calcific Aortic Valve Disease: Molecular Mechanisms and Therapeutic Approaches. Eur Cardiol. 2015 Winter; 10(2):108-112.
Libby, P. et al. Atherosclerosis. Nat Rev Dis Primers 5, 56 (2019). https://doi.org/10.1038/s41572-019-0106-z
Loria, Roger, M., US 2001/0014675 A1
Lusis AJ, Mar R, Pajukanta P. Genetics of atherosclerosis. Annu Rev Genomics Hum Genet 2004; 5: 189-218.
Magyar et al.: Increased Total Scavenger Capacity and Decreased Liver Fat Content in Rats Fed Dehydro-epiandrosterone and Its Sulphate on a High-Fat Diet Gerontology 2011; 57, 343-349
Mathew, R. O., Bangalore, S., Lavelle, M. P., Pellikka, P. A., Sidhu, M. S., Boden, W. E., and Asif, A. (2017). Diagnosis and management of atherosclerotic cardiovascular disease in chronic kidney disease: a review. Kidney International, 91(4), 797-807.
Medical Encyclopedia, Retinal artery occlusion. Retrieved from https://medlineplus.gov/ency/article/001028.htm.
Mohler ER, 3rd, Gannon F, Reynolds C, et al. Bone formation and inflammation in cardiac valves. Circulation 2001; 103: 1522-1528.
Mohler ER, 3rd. Mechanisms of aortic valve calcification. Am J Cardiol 2004; 94: 1396-1402, a1396.
Naftolin F et al. Sex Steroids Block the Initiation of Atherosclerosis. Reproductive Sciences 2016, Vol. 23(12) 1620-1625
National Research Council (US) Committee on Diet and Health. (1989). Diet and Health: Implications for Reducing Chrinic Disease Risk. National Academy Press, Washington D.C., 1989
Olechnowicz-Tietz, S., Gluba, A., Paradowska, A., Banach, M., & Rysz, J. (2013). The risk of atherosclerosis in patients with chronic kidney disease. International urology and nephrology, 45(6), 1605-1612.
Perridon BW, Leuvenink HGD, Hillebrands J-L, et al. The role of hydrogen sulfide in aging and age-related pathologies. Aging 2016; 8: 2264-2289.
Potor L, Nagy P, Mehes G, et al. Hydrogen Sulfide Abrogates Hemoglobin-Lipid Interaction in Atherosclerotic Lesion. Oxid Med Cell Longev 2018; 2018: 3812568.
Roher, A. E., Esh, C., Kokjohn, T. A., Kalback, W., Luehrs, D. C., Seward, J. D., Sue, L. I., and Beach, T. G. (2003). Circle of Willis Atherosclerosis Is a Risk Factor for Sporadic Alzheimer's Disease. Arteriosclerosis, Thrombosis, and Vascular Biology. 2003; 23:2055-2062.
Sánchez-Guijo, A., Oji, V., Hartmann, M. F., Traupe, H., & Wudy, S. A. (2015). Simultaneous quantification of cholesterol sulfate, androgen sulfates, and progestagen sulfates in human serum by LC-MS/MS. Journal of lipid research, 56(9), 1843-1851.
Schauer JE, Schelin A, Hanson P, Stratman FW. Dehydroepiandrosterone and a b-agonist, energy transducers, alter antioxidant enzyme systems: influence of chronic training and acute exercise in rats. Arch Biochem Biophys 1990; 283, 503-11
Sikura KE, Potor L, Szerafin T, et al. Hydrogen sulfide inhibits calcification of heart valves; implications for calcific aortic valve disease. Br J Pharmacol 2019.
Singh, S. B., & Lin, H. C. (2015). Hydrogen Sulfide in Physiology and Diseases of the Digestive Tract. Microorganisms, 3(4), 866-889.
Song, C, Hiipakka, RA., Liao S. Auto-oxidized cholesterol sulfates are antagonistic ligands of liver X receptors: implications for the development and treatment of atherosclerosis. Steroids 2001; 66 473-479.
Speer MY, Giachelli CM. Regulation of cardiovascular calcification. Cardiovasc Pathol 2004; 13: 63-70.
Stary H C. et al. A definition of advanced types of atherosclerotic lesions and a histological classification of atherosclerosis. A report from the Committee on Vascular Lesions of the Council on Arteriosclerosis, American Heart Association. Circulation. 1995 Sep 1;92(5):1355-74.
Strickley, R.G. Solubilizing Excipients in Oral and Injectable Formulations. Pharmaceutical Research, Vol. 21, No. 2, February 2004
U.S. Department of Health and Human Services, Food and Drug Administration, Center for Drug Evaluation and Research (2005). Guidance for Industry - Estimating the Maximum Safe Starting Dose in Initial Clinical Trials for Therapeutics in Adult Healthy Volunteers
Wang R. Two's company, three's a crowd: can H2S be the third endogenous gaseous transmitter? Faseb j 2002; 16: 1792-1798.
Yutzey KE, Demer LL, Body SC, et al. Calcific aortic valve disease: a consensus summary from the Alliance of Investigators on Calcific Aortic Valve Disease. Arterioscler Thromb Vasc Biol 2014; 34: 2387-2393.
Zhan J-Q, Zheng L-L, Chen H-B, et al. Hydrogen Sulfide Reverses Aging-Associated Amygdalar Synaptic Plasticity and Fear Memory Deficits in Rats. Frontiers in Neuroscience 2018; 12.
Zhang, Y., Tang, Z. H., Ren, Z., Qu, S. L., Liu, M. H., Liu, L. S., & Jiang, Z. S. (2013). Hydrogen sulfide, the next potent preventive and therapeutic agent in aging and age-associated diseases. Molecular and cellular biology, 33(6), 1104-1113. https://doi.org/10.1128/MCB.01215-12

## Claims

1. A compound according to formula (I), 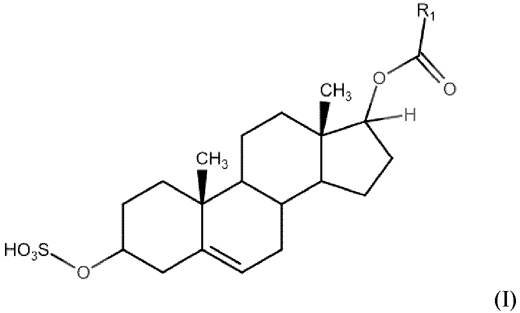 wherein R₁ is C₇-C₁₃ alkyl,
or any salt or solvate thereof, preferably any pharmaceutically acceptable salt thereof.

2. The compound according to claim 1, wherein R₁ is selected from the group consisting of C₉-C₁₃ alkyl, C₇-C₁₁ alkyl, C₁₁-C₁₃ alkyl, wherein preferably each alkyl is a linear alkyl.

3. The compound according to claim 1 or 2, wherein R₁ is a linear alkyl.

4. The compound according to any of claims 1 to 3, wherein R₁ is C₁₁ alkyl.

5. The compound according to any of claims 1 to 4, wherein the compound is 17-lauroyloxy-5-androstene-3-sulphate, and wherein preferably the salt is a sodium salt.

6. The compound according to any of claims 1 to 5 for use in the treatment of arteriosclerosis, in particular atherosclerosis.

7. The compound for use according to claim 6 for use in the therapeutic treatment of arteriosclerosis, preferably atherosclerosis, in particular wherein the formation of atherosclerotic plaques in arteries of a subject is reversed.

8. The compound for use according to claim 6 for use in the prevention of arteriosclerosis, preferably atherosclerosis, in particular for use in the prevention of or in reducing or in alleviating the formation of atherosclerotic lesions in arteries or atherosclerotic plaques in arteries.

9. The compound according to any of claims 1 to 5 for use in reducing the amount of reactive oxygen species (ROS) and/or inhibiting ROS production; thereby reducing oxidative stress and preventing the onset of or alleviating oxidative stress diseases.

10. The compound for use according to any of claims 6 to 9 for use in the therapy of or in the prevention of one or more of the following conditions:
- thrombosis,
- coronary heart disease or coronary artery disease, including stenosis of the coronary arteries,
- myocardial infarction or heart attack or consequences thereof,
- stroke, in particular ischemic stroke and/or hemorrhagic stroke,
- thrombosis, e.g. thrombosis formed over an atherosclerotic plaque,
- angina pectoris,
- peripheral arterial disease,
- aortic calcification, like calcific aortic valve disease (CAVD),
- atherosclerosis in renal dysfunctions like chronic kidney disease (CKD),
- atherosclerosis in neurodegenerative diseases.

11. A pharmaceutical composition or medicament comprising a compound selected from the group consisting of compounds according to any of claims 1 to 5 or any pharmaceutically acceptable salt thereof,
as well as a pharmaceutically acceptable carrier or excipient.

12. The pharmaceutical composition or medicament according to claim 11, which is for oral administration, wherein preferably the pharmaceutical composition or medicament is present in the form of tablets for oral administration.

13. The pharmaceutical composition or medicament according to claim 11, wherein the pharmaceutical composition or medicament is for administration to a subject or patient intravenously.

14. The pharmaceutical composition or medicament according to any of claims 11 to 13, wherein the pharmaceutical composition or medicament is formulated as a daily dose of 1 to 500 mg/day, preferably 5 to 200 mg/day or the pharmaceutical composition or medicament comprises a dose of 1-200 mg/day.

15. The pharmaceutical composition or medicament according to any of claims 11 to 14 for use in a condition as defined in any of claims 6 to 10.

## Patentansprüche

1. Verbindung gemäß Formel (I), 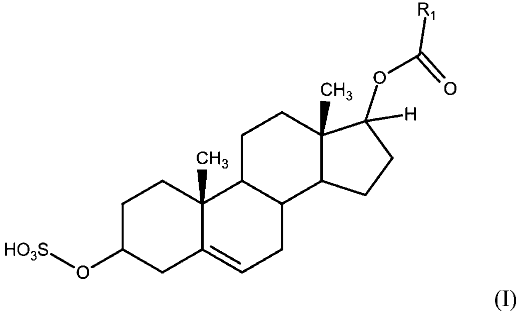 wobei R₁ C₇-C₁₃-Alkyl ist,
oder ein beliebiges Salz oder Solvat davon, vorzugsweise ein beliebiges pharmazeutisch verträgliches Salz davon.

2. Verbindung nach Anspruch 1, wobei R₁ aus der Gruppe ausgewählt ist, die aus C₉-C₁₃-Alkyl, C₇-C₁₁-Alkyl, C₁₁-C₁₃-Alkyl besteht, wobei vorzugsweise jedes Alkyl ein lineares Alkyl ist.

3. Verbindung nach Anspruch 1 oder 2, wobei R₁ ein lineares Alkyl ist.

4. Verbindung nach einem der Ansprüche 1 bis 3, wobei R₁ C₁₁-Alkyl ist.

5. Verbindung nach einem der Ansprüche 1 bis 4, wobei die Verbindung 17-Lauroyloxy-5-androsten-3-sulfat ist und wobei das Salz vorzugsweise ein Natriumsalz ist.

6. Verbindung nach einem der Ansprüche 1 bis 5 zur Verwendung bei der Behandlung von Arteriosklerose, insbesondere Atherosklerose.

7. Verbindung zur Verwendung nach Anspruch 6 zur Verwendung bei der therapeutischen Behandlung von Arteriosklerose, vorzugsweise Atherosklerose, insbesondere wobei die Bildung von atherosklerotischen Plaques in den Arterien eines Patienten umgekehrt wird.

8. Verbindung zur Verwendung nach Anspruch 6 zur Verwendung bei der Vorbeugung von Arteriosklerose, vorzugsweise Atherosklerose, insbesondere zur Verwendung bei der Vorbeugung oder Verringerung oder Linderung der Bildung atherosklerotischer Läsionen in Arterien oder der Bildung atherosklerotischer Plaques in Arterien.

9. Verbindung nach einem der Ansprüche 1 bis 5 zur Verwendung bei der Verringerung der Menge reaktiver Sauerstoffspezies (ROS) und/oder der Hemmung der ROS-Produktion; dadurch wird oxidativer Stress verringert und der Ausbruch von durch oxidativen Stress verursachten Krankheiten verhindert oder gelindert.

10. Verbindung zur Verwendung nach einem der Ansprüche 6 bis 9 zur Verwendung bei der Therapie oder Vorbeugung einer oder mehrerer der folgenden Erkrankungen:
- Thrombose,
- Koronare Herzkrankheit oder Koronararterienerkrankung, einschließlich Stenose der Koronararterien,
- Myokardinfarkt oder Herzanfall oder die Folgen davon,
- Schlaganfall, insbesondere ischämischer Schlaganfall und/oder hämorrhagischer Schlaganfall,
- Thrombose, z. B. Thrombose, die sich über einer atherosklerotischen Plaque gebildet hat,
- Angina Pectoris,
- periphere arterielle Krankheit,
- Aortenverkalkung, wie kalzifizierte Aortenklappenerkrankung (CAVD),
- Atherosklerose bei Nierenfunktionsstörungen wie chronischer Nierenerkrankung (CKD),
- Atherosklerose bei neurodegenerativen Erkrankungen.

11. Pharmazeutische Zusammensetzung oder Medikament, das eine Verbindung ausgewählt aus der Gruppe bestehend aus Verbindungen gemäß einem der Ansprüche 1 bis 5 oder ein pharmazeutisch verträgliches Salz davon sowie einen pharmazeutisch verträglichen Träger oder Hilfsstoff enthält.

12. Pharmazeutische Zusammensetzung oder Medikament nach Anspruch 11 zur oralen Verabreichung, wobei die pharmazeutische Zusammensetzung oder das Medikament vorzugsweise in Form von Tabletten zur oralen Verabreichung vorliegt.

13. Pharmazeutische Zusammensetzung oder Medikament gemäß Anspruch 11, wobei die pharmazeutische Zusammensetzung oder das Medikament zur intravenösen Verabreichung an einem Subjekt oder einen Patienten bestimmt ist.

14. Pharmazeutische Zusammensetzung oder Medikament gemäß einem der Ansprüche 11 bis 13, wobei die pharmazeutische Zusammensetzung oder das Medikament als Tagesdosis von 1 bis 500 mg/Tag, vorzugsweise 5 bis 200 mg/Tag formuliert ist oder die pharmazeutische Zusammensetzung oder das Medikament eine Dosis von 1.200 mg/Tag umfasst.

15. Pharmazeutische Zusammensetzung oder Medikament gemäß einem der Ansprüche 11 bis 14 zur Verwendung bei einem Zustand, wie er in einem der Ansprüche 6 bis 10 definiert ist.

## Revendications

1. Composé selon la formule (I), 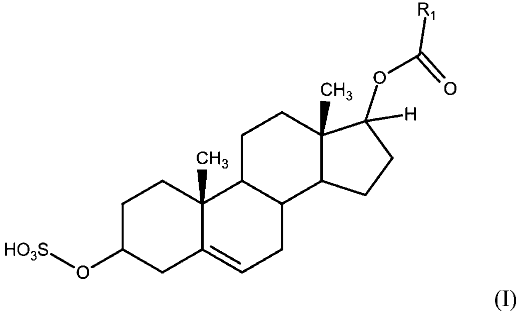 dans laquelle R₁ représente un groupe alkyle en C₇-C₁₃,
ou un de ses sels ou solvates, de préférence un de ses sels pharmaceutiquement acceptables.

2. Composé selon la revendication 1, dans lequel R₁ est choisi dans le groupe constitué par un groupe alkyle en C₉-C₁₃, un groupe alkyle en C₇-C₁₁, un groupe alkyle en C₁₁-C₁₃, dans lequel chaque alkyle est de préférence un alkyle linéaire.

3. Composé selon la revendication 1 ou 2, dans lequel R₁ représente un groupe alkyle linéaire.

4. Composé selon l'une quelconque des revendications 1 à 3, dans lequel R₁ représente un groupe alkyle en C₁₁.

5. Composé selon l'une quelconque des revendications 1 à 4, dans lequel le composé est le 17-lauroyloxy-5-androstène-3-sulfate, et dans lequel le sel est de préférence un sel de sodium.

6. Composé selon l'une quelconque des revendications 1 à 5, pour une utilisation dans le traitement de l'artériosclérose, plus particulièrement de l'athérosclérose.

7. Composé pour une utilisation selon la revendication 6, pour une utilisation dans le traitement thérapeutique de l'artériosclérose, de préférence de l'athérosclérose, en particulier où la formation de plaques d'athérosclérose dans les artères d'un sujet est inversée.

8. Composé pour une utilisation selon la revendication 6, pour une utilisation dans la prévention de l'artériosclérose, de préférence de l'athérosclérose, en particulier pour une utilisation dans la prévention ou la réduction ou l'atténuation de la formation de lésions d'athérosclérose ou de plaques d'athérosclérose dans les artères.

9. Composé selon l'une quelconque des revendications 1 à 5, pour une utilisation dans la réduction de la quantité d'espèces réactives de l'oxygène (ERO) et/ou l'inhibition de la production d'ERO ; réduisant ainsi le stress oxydatif et prévenant l'apparition de maladies liées au stress oxydatif ou atténuant ces maladies.

10. Composé pour une utilisation selon l'une quelconque des revendications 6 à 9, pour une utilisation dans le traitement ou la prévention d'une ou plusieurs des affections suivantes :
- thrombose,
- maladie coronarienne ou coronaropathie, y compris la sténose des artères coronaires,
- infarctus du myocarde ou crise cardiaque, ou leurs séquelles,
- accident vasculaire cérébral, en particulier accident vasculaire cérébral ischémique et/ou hémorragique,
- thrombose, par exemple thrombose formée sur une plaque d'athérosclérose,
- angine de poitrine,
- artériopathie périphérique,
- calcification aortique, telle que la valvulopathie aortique calcifiée (VAC),
- athérosclérose associée à une insuffisance rénale, telle qu'une maladie rénale chronique (MRC),
- athérosclérose associée à des maladies neurodégénératives.

11. Composition pharmaceutique ou médicament comprenant un composé choisi dans le groupe constitué par des composés selon l'une quelconque des revendications 1 à 5, ou un de ses sels pharmaceutiquement acceptables,
ainsi qu'un excipient ou support pharmaceutiquement acceptable.

12. Composition pharmaceutique ou médicament selon la revendication 11, destiné à l'administration orale, où de préférence la composition pharmaceutique ou le médicament est présenté sous forme de comprimés pour administration orale.

13. Composition pharmaceutique ou médicament selon la revendication 11, où la composition pharmaceutique ou le médicament est destiné à être administré par voie intraveineuse à un sujet ou à un patient.

14. Composition pharmaceutique ou médicament selon l'une quelconque des revendications 11 à 13, où la composition pharmaceutique ou le médicament est formulé comme une dose quotidienne de 1 à 500 mg/jour, de préférence de 5 à 200 mg/jour, ou la composition pharmaceutique ou le médicament comprend une dose de 1 à 200 mg/jour.

15. Composition pharmaceutique ou médicament selon l'une quelconque des revendications 11 à 14 pour une utilisation dans une affection telle que définie dans l'une quelconque des revendications 6 à 10.
